# EUROPEAN PATENT SPECIFICATION

(11) **EP 3 463 120 B1**
(45) Date of publication and mention of the grant of the patent: **27.07.2022**
(21) Application number: 17739356.8
(22) Date of filing: 02.06.2017
(51) Int. Cl.: A61B 17/32, A61F 2/24, A61B 17/3203, A61B 17/34, A61B 17/22

(54) **DEVICES FOR MANIPULATING BLOOD VESSEL WALLS AND ASSOCIATED SYSTEMS**
VORRICHTUNGEN ZUR MANIPULATION VON BLUTGEFÄSSWÄNDEN SOWIE ZUGEHÖRIGE SYSTEME
ISPOSITIFS DE MANIPULATION DE PAROIS VASCULAIRES ET SYSTÈMES ASSOCIÉS

(30) Priority: 03.06.2016 US 201662345687 P; 14.11.2016 US 201662422019 P
(43) Date of publication of application: 10.04.2019
(73) Proprietor: Intervene, Inc., Mountain View, CA 94040 (US)
(72) Inventor: WILSON, Fletcher, T., Mountain View CA 94040 (US); BATTEN, David, Mountain View CA 94040 (US); CLARK, Benjamin, J., Mountain View CA 94040 (US); GARRISON, Michi, Mountain View CA 94040 (US); DELL, Kent, Mountain View CA 94040 (US); GEORGE, William, R., Mountain View CA 94040 (US)
(74) Representative: Forresters IP LLP
(86) International application number: PCT/US2017/035851
(87) International publication number: WO 2017/210656

(56) References cited:
- WO-A1-2011/106735
- US-A1- 2012 289 987
- US-A1- 2013 317 534
- US-A1- 2015 359 630

## Description

### CROSS-REFERENCE TO RELATED APPLICATION(S)

The present application claims the benefit of U.S. Provisional Patent Application No. 62/422,019, filed November 14, 2016, and U.S. Provisional Patent Application No. 62/345,687, filed June 3, 2016.

### TECHNICAL FIELD

The present technology relates generally to devices and exemplary methods for intravascular modification of body lumens. Some embodiments of the present technology relate to the intravascular creation of valve leaflets within blood vessels.

### BACKGROUND

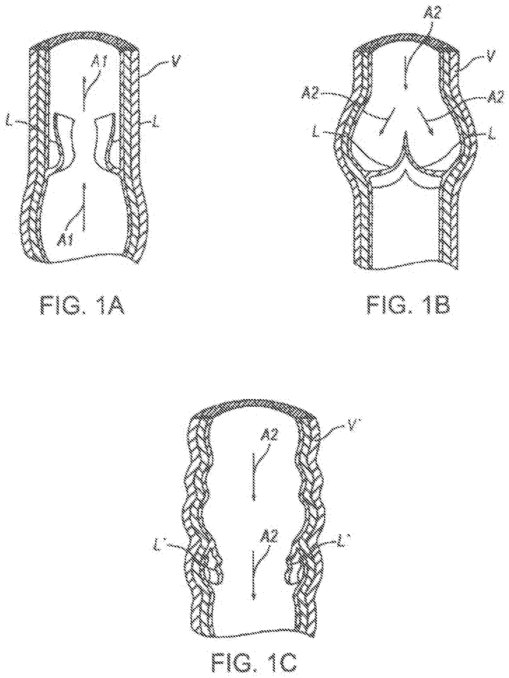

Figures 1A and 1B are schematic cross-sectional views of a normal human vein V. The vein V includes a valve formed of two leaflets L. Figure 1A shows the valve in an open position in which the leaflets L separate to allow blood to flow towards the heart in the direction indicated by arrows A1. Figure 1B shows the valve in a closed position in which the leaflets L come together to block the flow of blood away from the heart in the direction indicated by arrows A2. Figure 1C shows a vein V having a diseased or otherwise damaged valve comprised of leaflets L'. As shown in Figure 1C, the leaflets L' are structurally incompetent and allow venous reflux, or the flow of venous blood away from the heart (arrows A2). Venous reflux can lead to varicose veins, pain, swollen limbs, leg heaviness and fatigue, and skin ulcers, amongst other symptoms.

Venous reflux can occur anywhere throughout the venous system, which includes superficial veins (veins closer to the skin) and deep veins. Because deep veins are harder to access, deep veins are also harder to treat surgically. Existing methods for treating damaged or diseased vein valves in deep veins include surgical repair of the diseased vein and/or valve, removal of the damaged vein, and/or vein bypass. However, all of the foregoing treatment options include relatively lengthy recovery times and expose the patient to the risks involved in any surgical procedure, such as infection and clotting. Experimental treatments such as implantable venous valves, external venous valve banding, and heat-induced vein shrinkage have been attempted but each treatment has significant shortcomings. In addition, compression stockings are sometimes used to ameliorate symptoms but do not address the underlying problem.

US 2012/289987 discloses systems and methods for endoluminal valve creation.

Accordingly, there exists a need for improved devices and systems for treating damaged or diseased valves.

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present disclosure.
Figures 1A and 1B are schematic cross-sectional views of a normal human vein.
Figure 1C is a schematic cross-sectional view of an irregular human vein having a damaged or diseased valve.
Figures 2A-2D show various components of a valve formation system configured in accordance with the present technology.

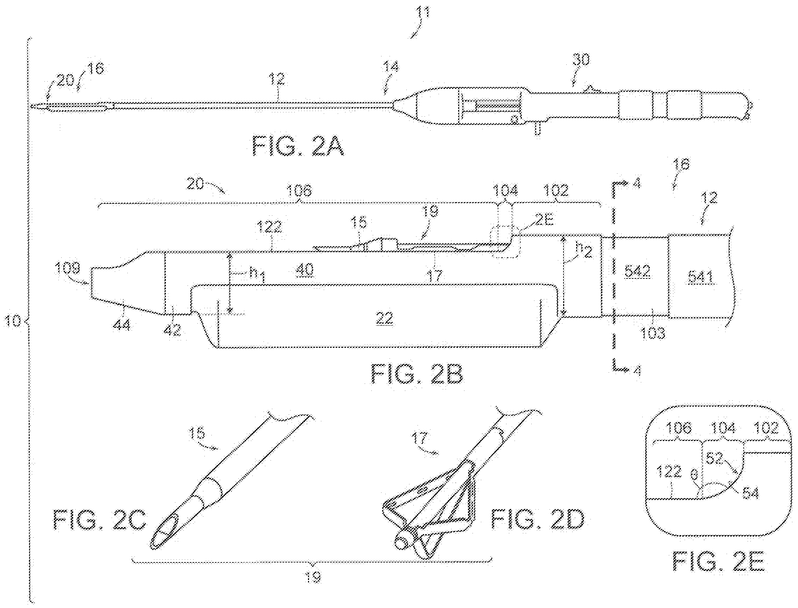

Figure 2A is a side view of a catheter assembly of the valve formation system configured in accordance with the present technology.
Figure 2B is an enlarged view of the distal portion of the catheter assembly in Figure 2A configured in accordance with the present technology.
Figure 2C is an isometric view of the distal portion of a tissue penetration assembly configured in accordance with the present technology.
Figure 2D is an isometric view of the distal portion of a valve creation assembly configured in accordance with the present technology.
Figure 2E is an enlarged view of a portion of the valve creation assembly shown in Figure 2B.

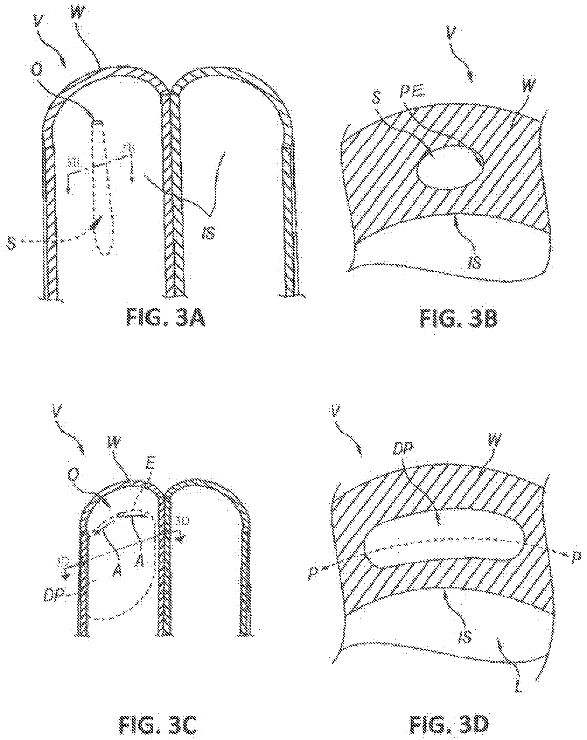

Figure 3A is a front-elevated, splayed view of a blood vessel showing an opening at an interior surface of the blood vessel wall and a space within the blood vessel wall.
Figure 3B is a cross-sectional end view of the space shown in Figure 3A.
Figure 3C is a front-elevated, splayed view of the blood vessel in Figures 3A and 3B showing a dissection pocket within the blood vessel wall.
Figure 3D is a cross-sectional end view of the dissection pocket shown in Figure 3C.

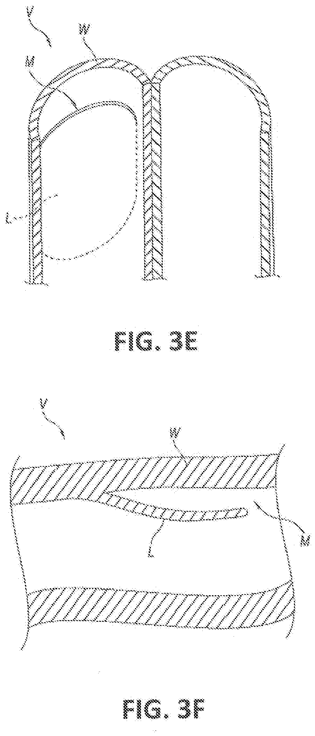

Figure 3E is a front-elevated, splayed view of the blood vessel in Figures 3A-3D, showing a leaflet formed of the blood vessel wall having a mouth.
Figure 3F is a side cross-sectional view showing the leaflet of Figure 3E.

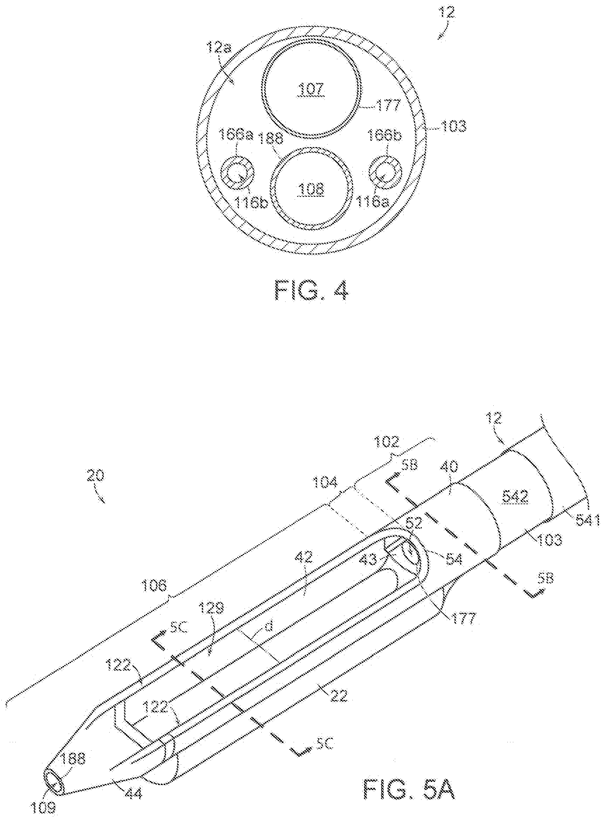

Figure 4 is a cross-sectional end view of the distal portion of the catheter assembly shown in Figure 2B, taken along line 4-4.

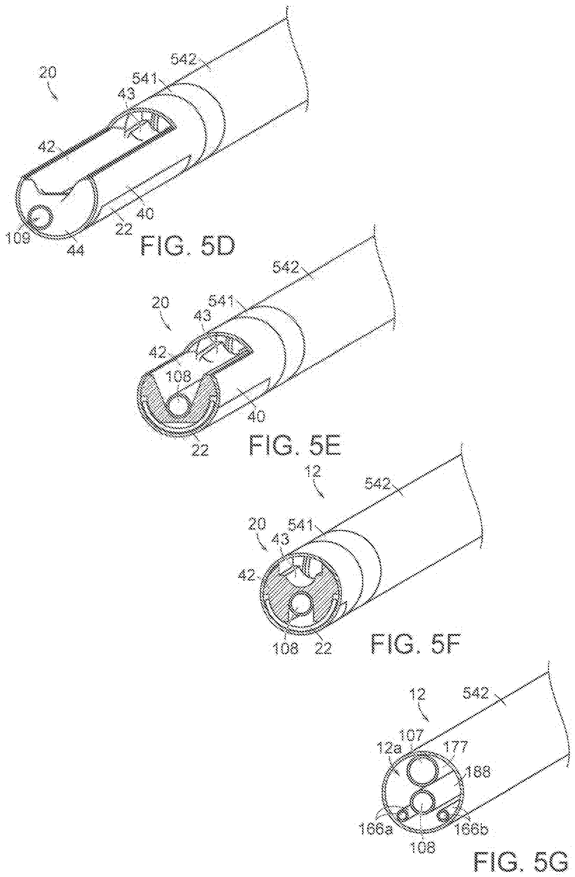

Figure 5A is an isometric view of the support assembly of the catheter assembly shown in Figures 2A and 2B configured in accordance with the present technology.

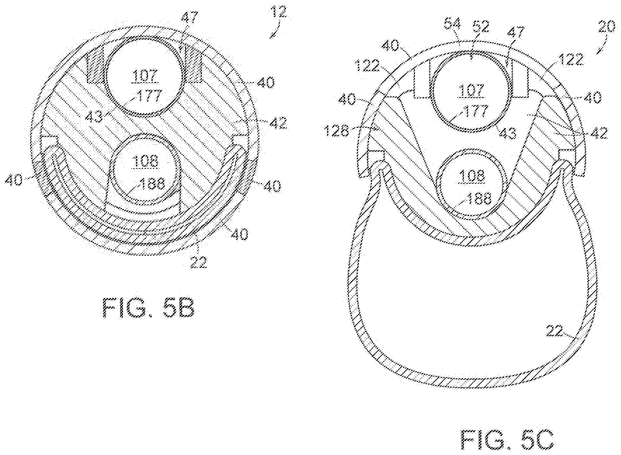

Figures 5B and 5C are cross-sectional end views of the support assembly shown in Figure 5A, taken along lines 5B-5B and 5C-5C, respectively.
Figure 5D is another isometric view of the support assembly, and Figures 5E-5G are cross-sectional end views taken at different locations along the axis of distal portion shown in Figure 5D.

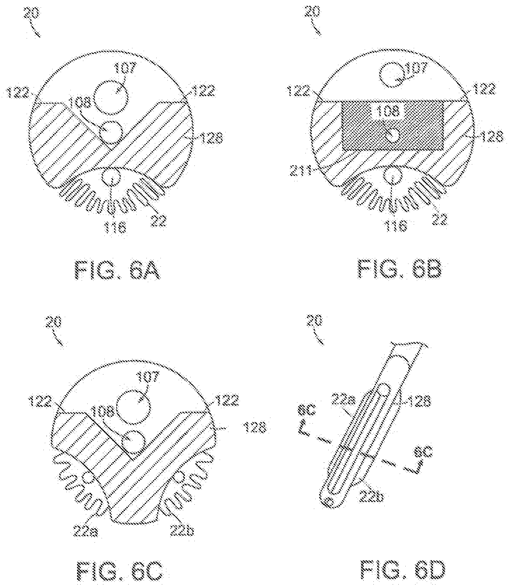

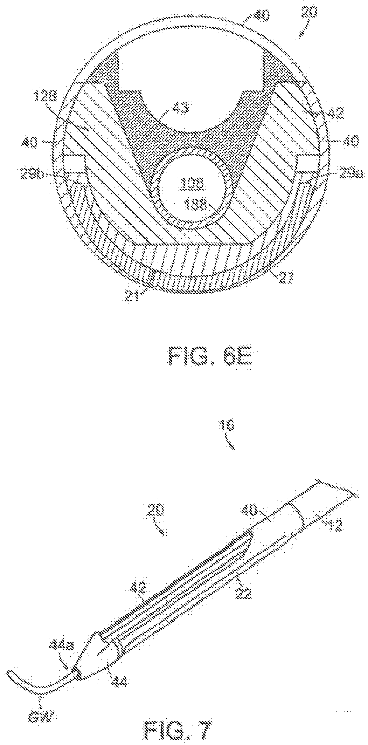

Figures 6A-6C and 6E are cross-sectional end views of different embodiments of support assemblies. Figure 6D is an isometric view of the distal portion shown in Figure 6C.
Figure 7 is an isometric view of a distal portion of a catheter assembly configured in accordance with the present technology.

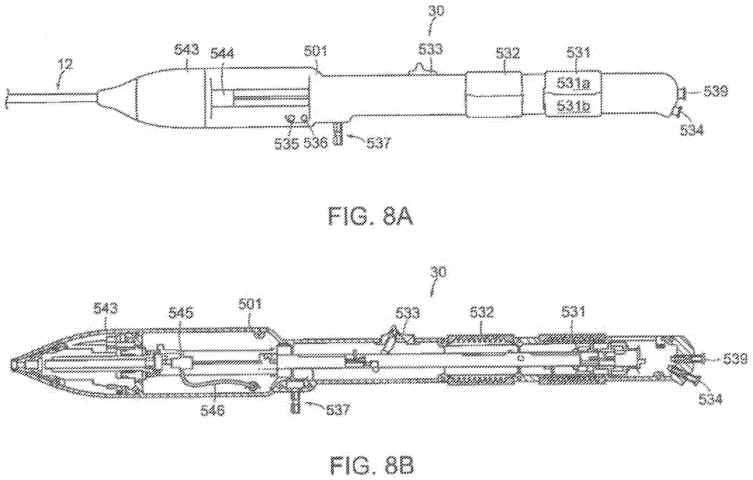

Figures 8A and 8B are side and side cross-sectional views of a handle assembly configured in accordance with the present technology.

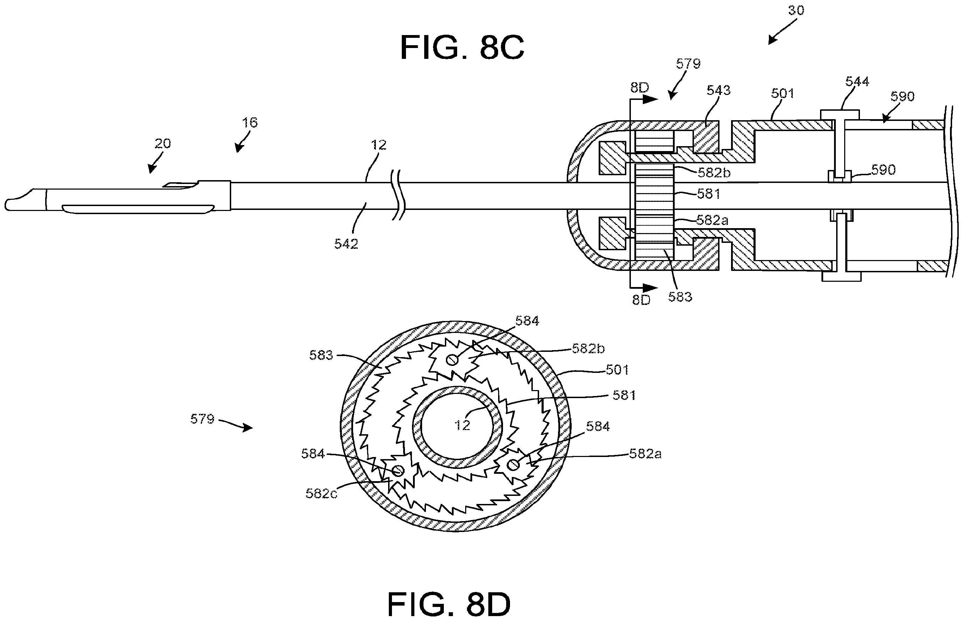

Figures 8C-8G show embodiments of a catheter assembly coupled to a handle assembly.

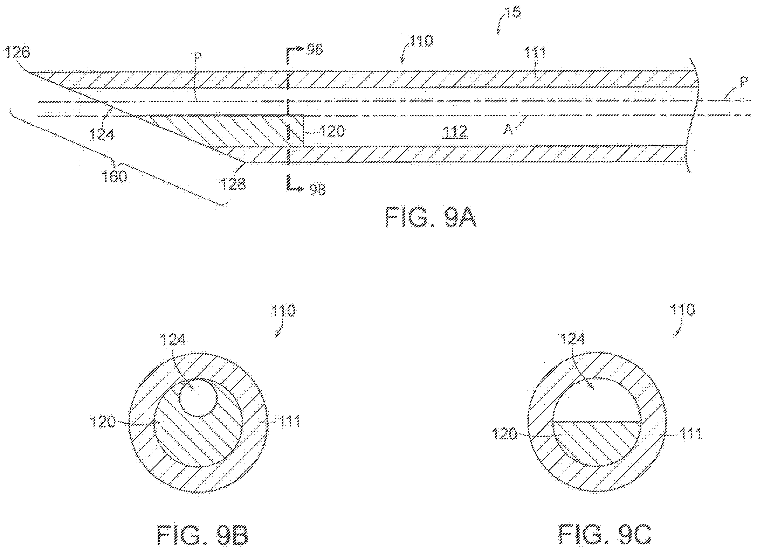

Figure 9A is a cross-sectional side view of a tissue penetration assembly configured in accordance with the present technology.
Figure 9B is a cross-sectional end view of the tissue penetration assembly shown in Figure 9A taken along 9B-9B.
Figure 9C is a cross-sectional end view of another embodiment of a tissue penetration assembly configured in accordance with the present technology.

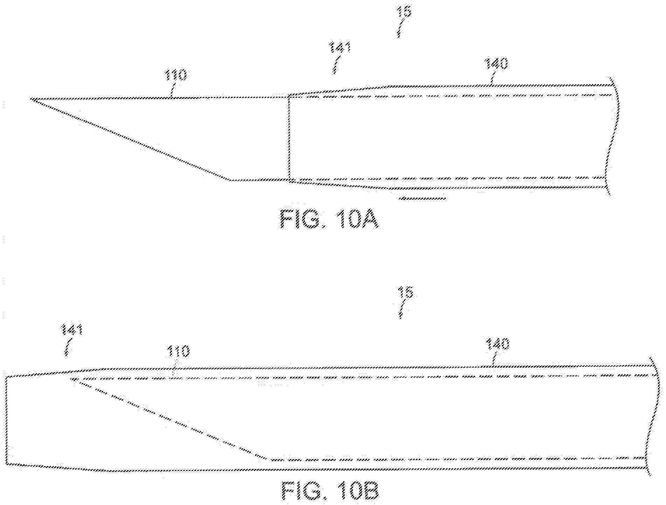

Figures 10A and 10B are side views of a tissue penetration assembly configured in accordance with the present technology.

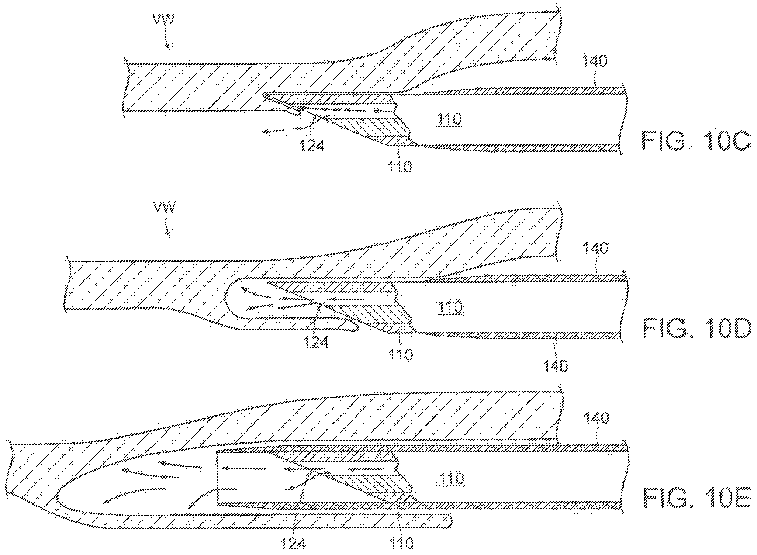

Figures 10C-10E illustrate a method of accessing an interior portion of a vessel wall utilizing the tissue penetration assembly in accordance with the present technology.

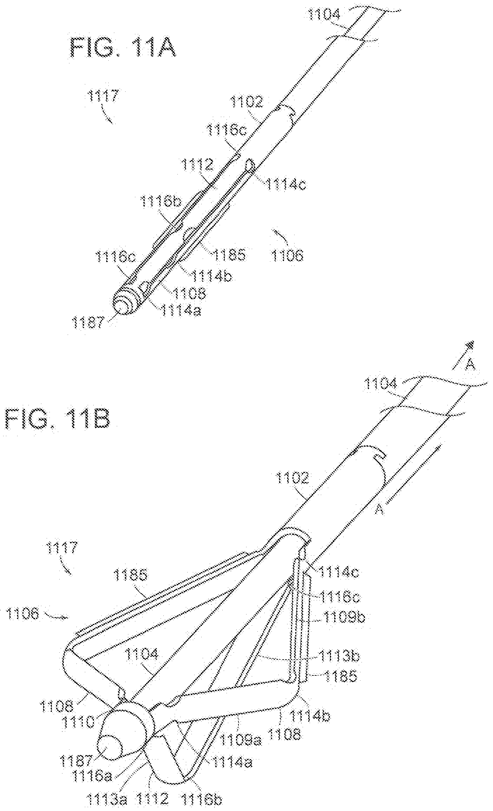

Figures 11A and 11B are isometric views of an embodiment of a valve creation assembly in accordance with the present technology, shown in a low-profile and deployed state, respectively.

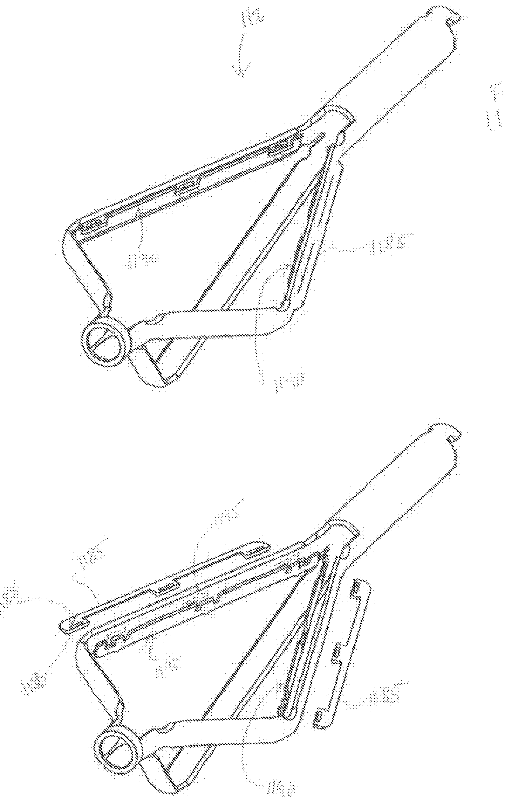

Figures 11C and 11D illustrate a method for attaching cutting elements to dissection arms of the valve creation assembly shown in Figures 11A and 11B.

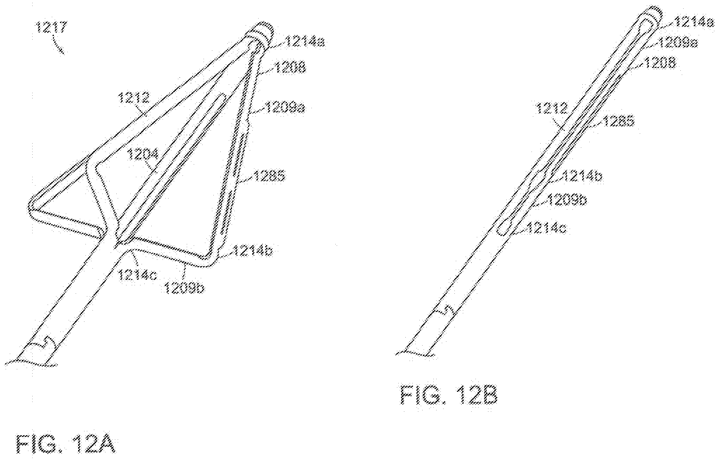

Figures 12A and 12B are isometric views of another embodiment of a valve creation assembly in accordance with the present technology, shown in a low-profile and deployed state, respectively.

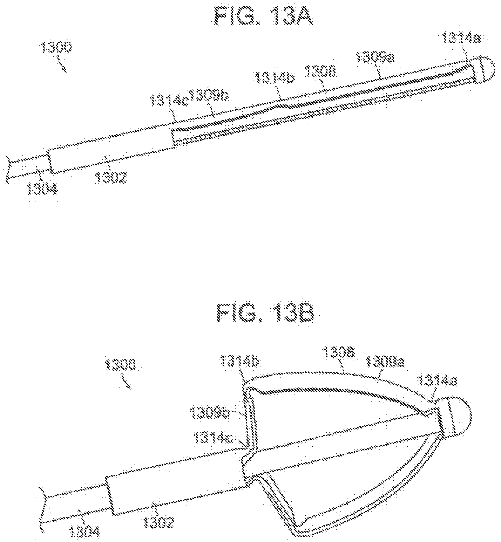

Figures 13A and 13B are views of a mechanical dissection device in an unexpanded sate and an expanded state, respectively.

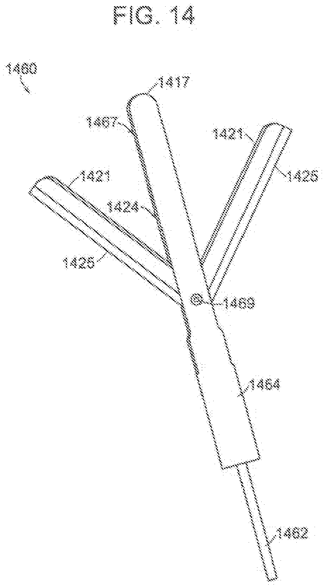

Figure 14 is an embodiment of a cutting device configured in accordance with the present technology.

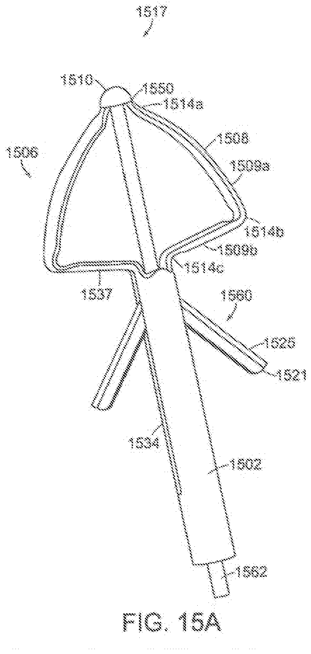

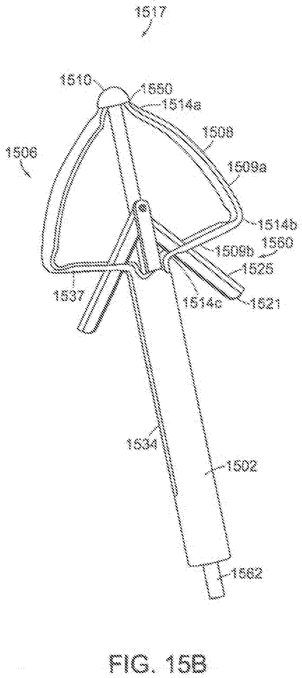

Figures 15A and 15B are views of an embodiment of a valve creation assembly with a slidably coupled dissection and cutting device, shown in two stages of dissection and cutting.

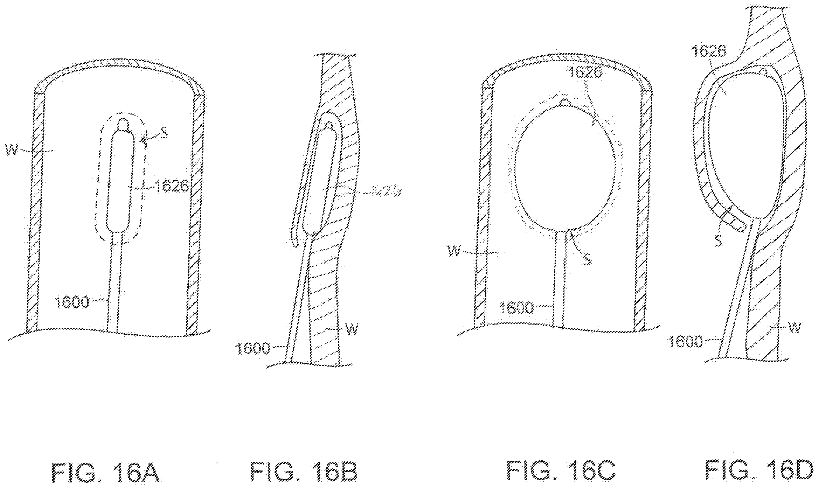

Figures 16A-16D are views of a balloon dissection device in an unexpanded state and an expanded state.

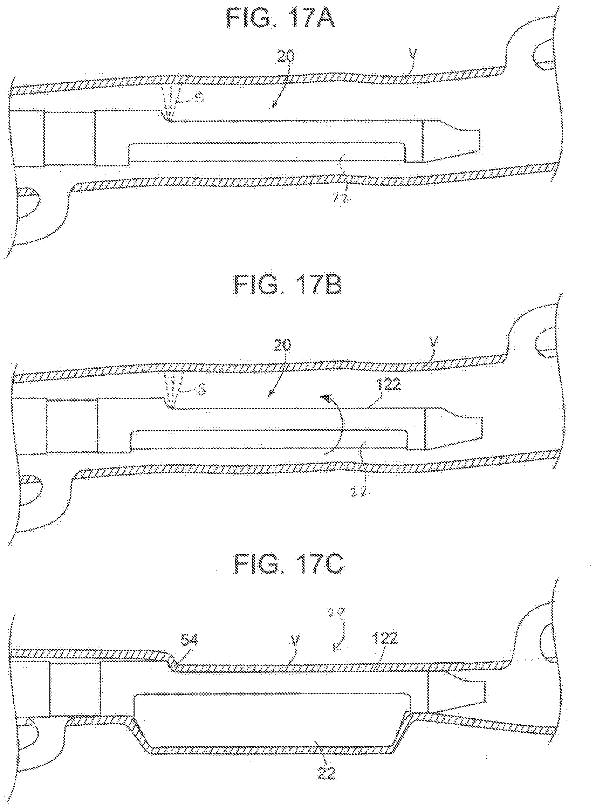

Figure 17A-17 illustrate a method of operation of a vessel layer access system in accordance with the present technology to access the layers of a vessel wall and form an autologous valve.

### DETAILED DESCRIPTION

The invention is defined by the appended claims. Methods of use and treatment are not part of the claimed invention.
1 inch = 25.4 mm.

The present technology provides devices, systems, and methods for gaining controlled access to tissue adjacent a body lumen, and for controlled dissection and manipulation of the accessed tissue to create one or more valve leaflets. An overview of the novel methodology of the present technology in conjunction with general aspects of one of the anatomical environments in which the disclosed technology operates is described below under heading 1.0 with reference to Figures 2A-3F. Particular embodiments of various subcomponents of the valve formation systems of the present technology are described below under headings 2.0-5.0. In particular, selected embodiments of catheter assemblies are described further under heading 2.0, selected embodiments of tissue penetration assemblies are described further under heading 3.0, and selected embodiments of valve creation assemblies are described below under heading 4.0. Representative methods for using the valve formation systems of the present technology to controllably access and dissect the interior portion of a blood vessel wall to create valve leaflets are described under heading 5.0.

With regard to the terms "distal" and "proximal" within this description, unless otherwise specified, the terms can reference a relative position of the portions of an catheter assembly and/or dissection device with reference to an operator and/or a location in the vasculature. 1.0 Overview

Figures 2A-2D show various components of an intravascular valve formation system 10 (also referred to herein as "system 10") configured to access an interior portion of a blood vessel wall from the true lumen of the blood vessel and dissect or otherwise separate two or more portions of a blood vessel wall to form one or more valve leaflets. As used herein, the term "separating two or more portions of a blood vessel wall" refers to the act of separating the vessel wall at least into a first layer and a second layer. The first layer can include intimal, medial, and/or adventitial tissue, and the second layer can include intimal, medial, and/or adventitial tissue. For example, dissection devices of the present technology can separate an intimal layer from a medial layer, a medial layer from an adventitial layer, a sub-medial layer from a sub-medial layer, an intimal and sub-medial layer from a sub-medial layer, etc.

As shown in Figures 2A-2D, the system 10 comprises a catheter assembly 11 (Figures 2A and 2B), a tissue penetration assembly 15 (Figure 2C), and a valve creation assembly 17 (Figure 2D). For ease of reference, the tissue penetration assembly 15 and the valve creation assembly 17 are referred to collectively herein as "dissection assemblies 19." In some embodiments, the system 10 includes more or fewer devices, assemblies, and/or other components. For example, the tissue penetration assembly and valve creation assembly may be a single assembly with tissue penetration and valve creation functionality.

The catheter assembly 11 includes an elongated shaft 12 configured to receive the dissection assemblies 19 therethrough. The dissection assemblies 19 may be delivered sequentially without exchanging components by sliding the valve creation assembly 17 over the tissue penetration assembly 15 (as shown in Figure 2B), or the dissection assemblies 19 may be delivered separately by exchanging one component for another. The shaft 12 has a proximal portion 14 and a distal portion 16. The catheter assembly 11 further includes a support assembly 20 carried by or affixed to the distal portion 16 of the shaft 12, as shown in the enlarged view of the distal portion 16 in Figure 2B. The support assembly 20 is configured to be positioned adjacent a blood vessel wall at a treatment site within a blood vessel lumen. As described in greater detail below under heading 2.0, the support assembly 20 includes an expandable member 22 (shown in an expanded state in Figures 2A and 2B) for stabilizing the support assembly 20 relative to the vessel wall at the treatment site. The support assembly 20 is configured to position and/or guide the dissection assemblies 19 delivered to the support assembly 20 at a specific orientation relative to the vessel wall. The catheter assembly 11 also includes a handle assembly 30 coupled to the proximal portion 14 of the shaft 12 and configured to be positioned external to the patient while the distal portion 16 of the shaft 12 is positioned intravascularly at the treatment site. The handle assembly 30 may include one or more fluid lines, one or more access ports for receiving the dissection assemblies 19, and one or more actuation elements for controlling and/or actuating one or more of the dissection assemblies 19 and/or components of the support assembly 20 (e.g., the expandable member 22), as described in greater detail below under major heading 2.0 and associated sub-headings.

Figures 3A-3F are schematic, splayed views of a blood vessel V (e.g., a vein) showing the interior of the blood vessel V during various stages of a method for the intravascular creation of a dissection pocket DP and/or a valve leaflet from a blood vessel wall W using the valve formation system 10 of the present technology. To begin, the catheter assembly 11 is intravascularly inserted over a guidewire (e.g., a 0.89 mm (0.035") guidewire) into a blood vessel, and the distal portion 16 is positioned adjacent the vessel wall at a treatment site within the blood vessel lumen. The expandable member 22 is expanded to position the support assembly 20 in apposition with an inner surface of the blood vessel wall. With reference to Figures 3A and 3B, the tissue penetration assembly 15 is then delivered as guided by the support assembly 20 towards the apposed vessel wall W and creates an opening O in the inner surface IS of the vessel wall W to gain access to an interior portion of the vessel wall W. During this stage, the tissue penetration assembly 15 creates an access space S within the vessel wall W for the subsequent delivery of the valve creation assembly 17. The tissue penetrating element may be connected to a pressurized fluid source to provide a hydrodissection force to assist in the creation of space S. Creation of the opening O and/or space S may also be achieved using other tissue penetrating assemblies, such as one or more of the dissection assemblies and/or inner members disclosed in U.S. Patent Application No. 14/667,670, filed March 24, 2015, U.S. Patent Application No. 13/035,752, filed February 25, 2011, U.S. Patent Application No. 13/450,432, filed April 18, 2012, and U.S. Patent Application No. 14/377,492, filed August 7, 2014.

Next, the valve creation assembly 17 is delivered through the catheter shaft 12 and support assembly 20 into the space S. Once the valve creation assembly 17 is positioned within the space S, the expandable member 22 of the support assembly 20 is collapsed and the entire support assembly is pulled back to provide more area in the vessel for the valve creation step. The valve creation assembly 17 is then actuated to separate tissue at the periphery PE (Figure 3B) of the space S. As shown in Figures 3A-3D, the enlarged space S forms a dissection pocket DP having a predetermined size and shape and extending along a dissection plane P within the vessel wall W.

To transform the dissection pocket DP into a valve leaflet L (shown in Figures 3E and 3F), the valve creation assembly 17 is used to cut the tissue at the proximal edge E of the dissection pocket DP adjacent the opening O to create a mouth M. For example, the valve creation assembly 17 can cut the vessel wall tissue at the edge of the dissection pocket DP that extends laterally away from the opening O, as indicated by arrows A in Figure 3C. In some embodiments, other suitable valve creation assemblies and/or separate cutting devices can be used to create the valve leaflet, such as those disclosed in U.S. Patent Application No. 14/667,670, filed March 24, 2015, U.S. Patent Application No. 13/035,752, filed February 25, 2011, U.S. Patent Application No. 13/450,432, filed April 18, 2012, and U.S. Patent Application No. 14/972,006, filed December 16, 2015.

It will be appreciated that the foregoing description is intended as a reference as and does not limit the description of the present technology presented herein.

### 2.0 Selected Embodiments of Catheter Assemblies

### 2.1 Selected Embodiments of Catheter Shafts and Distal Assemblies

Figure 4 is a cross-sectional end view of the shaft 12 taken along line 4-4 in Figure 2B. The dissection assemblies 19 are not shown in Figure 4 for ease of illustration. Referring to Figures 2B and 4 together, the shaft 12 may include a tubular sidewall 103 enclosing an interior region 12a (Figure 4), a first shaft 177 defining a first lumen 107 therethrough, a second shaft 188 defining a second lumen 108 therethrough, and third and fourth shafts 166a, 166b defining third and fourth and lumens 116a and 116b therethrough. Each of the first, second, third, and further shafts extend through the interior region 12a of the shaft 12. Each of the lumens 107, 108, 116a and 116b terminate proximally at corresponding ports at the handle assembly 30. In some embodiments, the shaft 12 does not include one or more of the shafts 177, 188, 166a and 166b and instead the shaft 12 may be molded or formed such that one or more of the lumens 107, 108, 116a, and 116b lumens are defined by openings in the materials of the shaft 12.

In some embodiments, the catheter shaft 12 of catheter assembly 11 is configured to allow access to a valve creation site in the femoral or popliteal veins from a common femoral vein access site. In such embodiments, the catheter shaft 12 has a working length of from about 50 cm to about 65 cm. In some embodiments, the catheter shaft 12 has a working length of from about 55 cm to about 60 cm. In some embodiments, the catheter shaft 12 is configured to allow access of a valve creation site in the femoral or popliteal veins from an internal jugular vein access site. In such embodiments, the catheter shaft 12 has a working length of from about 100 cm to about 130 cm. In some embodiments, the catheter shaft 12 has a working length of from about 110 cm to about 115 cm.

The first lumen 107 may be defined by the first shaft 177 and extends distally from the handle assembly 30 to an exit port 52 at the support assembly 20. The first lumen 107 is configured to slideably receive one or more devices therethrough (such as the tissue penetration assembly 15 and/or the valve creation assembly 17) and guide the received devices from the handle assembly 30 to the exit port 52. The first lumen 107 may also be configured such that one or both dissection assemblies exit the exit port 52 substantially parallel to a longitudinal axis of the support assembly 20 and/or a tissue engaging surface 122 of the support assembly 20, as discussed in greater detail below.

The second lumen 108 may be defined by the second shaft 188 and extends distally from the handle assembly 30 to an opening 109 at a distal terminus of the support assembly 20. The second lumen 108 is configured to slideably receive a guidewire therethrough (e.g., an 0.89 mm (0.035") guidewire) during delivery of the distal portion 16 to a treatment site within a blood vessel. The second lumen 108 is also configured to slideably receive a visualization device (not shown) therethrough for visualization of the treatment site. Examples of visualization devices include an intravascular ultrasound (IVUS) catheter, an angioscope, an optical coherence tomography (OTC) device, and/or other imaging catheters. In some embodiments, the shaft 12 includes a guidewire lumen and a separate visualization lumen. In yet another embodiment, the shaft 12 does not include a lumen for receiving a guidewire and/or a visualization device therethrough.

The third and fourth lumens 116a, 116b may be defined by elongated tubes 166a, 166b, respectively, that extend distally from the handle assembly 30 and terminate at the support assembly 20. In some embodiments, the openings at the end of the tubes are generally axially aligned with a proximal end portion of the expandable member 22. The third and fourth lumens 116a, 116b can be inflation lumens that fluidly connect a pressurized fluid source (e.g., a syringe, a pump, etc.) to an interior portion of the expandable member 22. In some embodiments, the shaft 12 may include more or fewer inflation lumens (e.g., one inflation lumen, three inflation lumens, four inflation lumens, etc.).

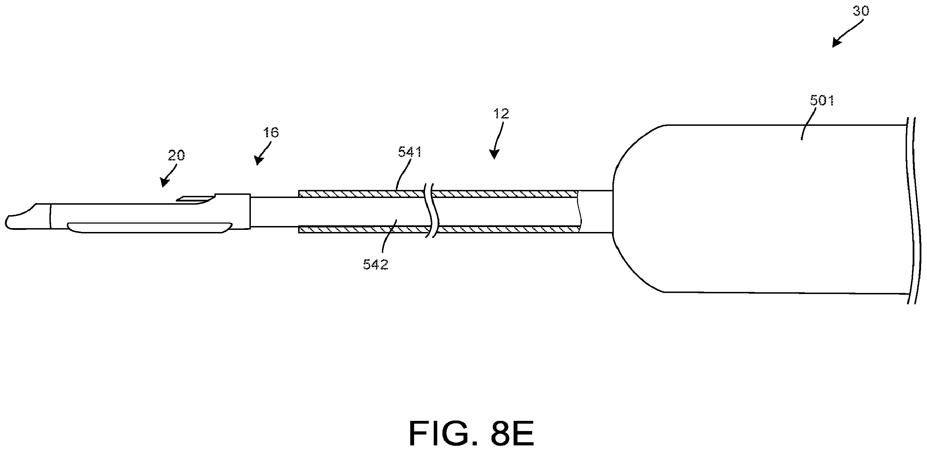

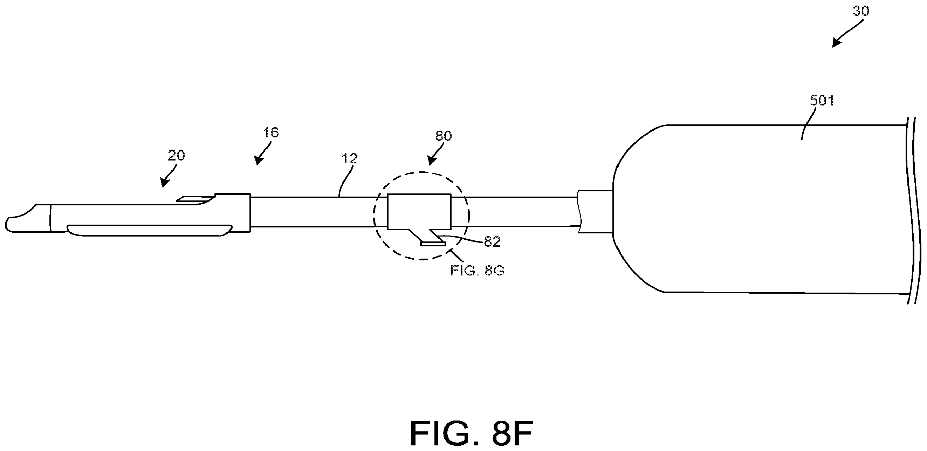

In some embodiments, the shaft 12 may be defined by a single tubular structure that encloses and/or defines one or more lumens. In the embodiment shown in Figure 2B, the shaft 12 includes an outer shaft 541 and an inner shaft 542, as described in greater detail below under sub-heading 2.3 and with reference to Figure 8D. In some embodiments, the shaft 12 includes an extension 80 along its proximal portion which may have a lumen that is fluidly coupled to the second lumen 108, as described in greater detail below under sub-heading 2.3 and with reference to Figures 8E and 8F.

The shaft 12 may be constructed from one or more flexible polymer materials such as Pebax^{®}, polyethylene, urethane, PVC, and/or blends thereof. The shaft 12 may contain lubricious additives to reduce friction as the shaft 12 rotates and translates with respect to the introducer sheath, or, in embodiments having an inner and outer shaft (such as outer and inner shafts 541 and 542 shown in Figure 8D), one shaft rotates and translates with respect to the other. Example additives include siloxane, PTFE, and the like. In some embodiments, the shaft 12 includes a lubricious layer, for example an inner FEP or PTFE liner. In order to accurately transfer rotational and translational forces from the handle assembly 30 to the support assembly 20 without being overly stiff, the shaft 12 may be a reinforced tubing, such as tubing reinforced with coiled or braided metal wire or ribbon layered between one or more polymer layers. Alternately, the shaft 12 may be constructed from cut metal or a stiff polymer hypotube configured such that the cut pattern provides the desired flexibility to the tubing without sacrificing the required rotational or translational strength.

### 2.2 Selected Embodiments of Support Assemblies

Figure 2E is an enlarged view of a portion of the support assembly 20 shown in Figure 2B. The dissection assemblies 19 are not shown in Figure 2E for ease of viewing the support assembly 20. Referring to Figures 2B and 2E, the support assembly 20 may include a first portion 102, a second portion 106, and an intermediate portion 104 extending between the first and second portions 102, 106. The second portion 106 can be distal to the intermediate portion 104, and the intermediate portion 104 can be distal to the first portion 102. The first portion 102 can have a greater cross-sectional area than the second portion 106, and the intermediate portion 104 can have a surface 54 that is angled or slanted radially inwardly (at an angle θ of from about 45 degrees to about 90 degrees) (see enlarged view of the support assembly 20 in Figure2E), in the direction of the second portion 106. In the embodiment shown in Figure 2B, the cross-sectional area of the support assembly 20 at any point along the length of the first portion 102 is greater than its cross-sectional area at any point along the length of the second portion 106.

In some embodiments, the support assembly 20 does not include an intermediate portion 104. In such embodiments, the first portion 102 transitions directly to the second portion 106 such that a portion of the outer surface of the support assembly 20 faces distally and is perpendicular to a longitudinal axis of the support assembly 20. Thus, reference below to the "slanted surface 52" is inclusive of the foregoing perpendicular surface configuration.

Figure 5A is an enlarged, isometric view of the support assembly 20 shown in Figure 2B. Figures 5B and 5C are cross-sectional end views taken along lines 5B-5B and 5C-5C in Figure 5A, respectively. Referring now to Figures 2B and 5A-5C, the illustrated embodiment of the support assembly 20 includes the expandable member 22, a support housing 40, a distal insert 42 positioned at least partially within the support housing 40, and a soft, atraumatic distal tip 44. The distal insert 42 sits within an elongated recess of the support housing 40, and the expandable member 22 and lumens 116a, 116b are sandwiched between the support housing 40 and the distal insert 42. In some embodiments, the support housing 40 and the distal insert 42 are a single component. In some embodiments, the support assembly 20 does not include a distal tip 44. The support housing 40 and the distal insert 42 (together or independently) include features that support guidance and stabilization of the dissection assemblies 19, as well as support positioning and maintaining the vessel wall in a desired orientation and position.

The support housing 40 can be a cut tube that supports and provides rigidity to the distal insert 42. The support housing 40 can be made of rigid tube materials such as, for example, stainless steel. In some embodiments, for example, the support housing 40 can be a cut stainless steel tube. The support housing 40 includes a sidewall defining an opening extending along at least a portion of the length of the sidewall. The portions of the sidewall on either side of the opening are separated by a distance d (Figure 5A). Along the second portion 106 of the support assembly 20, the distance d between the sidewalls on either side of the opening is relatively constant. Along the intermediate portion 104 of the support assembly 20, a height h₁ (Figure 2B) of the support assembly 20 increases in a proximal direction while the distance d between the sidewalls decreases in a proximal direction until reaching a proximal end of the opening and/or the first portion 102. (Height does not include the expandable member 22). The portion of the sidewall along the first portion 102 may have a height h₂ that is greater than the height h₁ along the second portion 106. The portion of the sidewall along the first portion 102 does not include the opening and instead has a closed, tubular shape. In the illustrated embodiment, the portion of the support housing 40 at the intermediate portion 104 forms the slanted surface 54. In some embodiments, at least a portion of the distal insert 42 forms the slanted surface 54. In yet other embodiments, at least a portion of the distal insert 42 and at least a portion of the support housing 40 form the slanted surface 54.

The supporting housing 40 can have other shapes, sizes, and configurations. For example, in some embodiments the height of the support housing 40 increases in a proximal direction along the intermediate portion 104 but the distance d between opposing sidewalls remains the same over that same length. In certain embodiments, the height of the support housing 40 and/or the distance between opposing portions of the support housing 40 can vary along the length of the first and second portions 102, 106.

The distal insert 42 may be made from one or more plastics and/or metals, such as polyether ether ketone ("PEEK"), polycarbonate ("PC"), polyetherimide ("PEI"), nylon, and/or other generally rigid materials. The materials may also include additives to increase rigidity, such as glass or carbon fiber. In the embodiment shown in Figures 2B and 5A, the portion of the distal insert 42 along the second portion 106 of the support assembly 20 forms a trough 128 (Figure 5A) having opposing sidewalls and an elongated recess 129 therebetween. Each of the sidewalls extend upwardly and terminate at tissue engaging surfaces 122 (also referred to as "surfaces 122"). The surfaces 122 of the sidewalls are configured to be positioned in apposition with an inner surface of the vessel wall when the support assembly 20 is positioned at a treatment site and the expandable member 22 is expanded against a circumferentially opposite portion of the vessel wall. Expansion of the expandable member 22 against the wall forces the surfaces 122 to contact the inner surface of the vessel wall, thereby conforming the vessel wall to the shape of the distal insert 42 and/or support housing 40. In the illustrated embodiment, the entirety of the surfaces 122 are generally flat and lie within a plane that is generally parallel to a longitudinal axis of the support assembly 20. In some embodiments, one or more portions of the surfaces 122 can be non-flat (e.g., include one or more protrusions extending therefrom) and/or may lie within a plane that is angled relative to the longitudinal axis of the support assembly 20. The distal insert 42 may also include a ledge portion 43, at least a portion of which is aligned with the intermediate portion 104 of the support assembly 20. The ledge portion 43 supports and/or guides the first shaft 177 and/or one or more components of the dissection assemblies 19.

In some embodiments, the length of the trough 128 is roughly the same or larger than the intended size of the leaflet to be created, as it defines the distance in which the tissue penetration assembly 15 and valve creation assembly 17 can be inserted into the tissue layers. In some embodiments, the length of the trough is between 20 and 40 millimeters. In some embodiments, the length of the trough is roughly 25-35 millimeters.

As best shown in Figure 5B, the surfaces 122 may lie along a plane that transects the exit port 52 of the device lumen 107. In the illustrated embodiment, the second lumen 108 is positioned below the first lumen 107 (or vice versa). In some embodiments, the second lumen 108 can be a separate tube extending through the trough 128 and terminating at or beyond a distal terminus of the support assembly 20.

The expandable member 22 can be an inflatable compliant balloon. Exemplary balloon materials include low durometer polyurethane, silicone, urethane-silicone blends, latex, and/or other polymeric elastomers. In some embodiments, the expandable member 22 may be positioned below the trough 128.

The support assembly 20 provides multiple functions during the valve creation procedure. For example, the support assembly 20 guides the dissection assemblies 19 to the target treatment site and positions the dissection assemblies 19 at the desired location and in the desired orientation relative to the vessel wall. The support assembly 20 also positions the vessel wall at a desired, known position and orientation relative to the exit port 52 and maintains the vessel wall in this position and orientation throughout some or all of the valve formation procedure. Another function of the support assembly 20 is to support one or more of the expandable member 22, an optional visualization device (and corresponding lumen), and a guidewire (and lumen).

The support assembly 20 may have other components and/or configurations. Examples of alternative support assembly embodiments are shown in Figures 6A-6E. In the embodiment shown in Figure 6A, the trough 128 has a generally triangular cross-sectional shape along the second portion 106. In some embodiments, the trough 128 can have parallel sidewalls (relative to one another) and a straight bottom, thereby defining an opening having a generally rectangular cross-sectional shape, as shown in Figure 6B. In a particular embodiment, the trough 128 has sidewalls angled towards one another and a straight bottom, thereby defining an opening having a generally trapezoidal cross-sectional shape. In some embodiments, the trough 128 may have a curved bottom portion such that the sidewalls define a u-shaped or semi-circular opening. In any of the trough embodiments disclosed herein, the edges of the sidewall at either side of the trough 128 may be rounded.

Figure 6B shows one embodiment of where some or all of the trough 128 contains an echolucent material 129. The second or visualization lumen 108 extends distally from the intermediate portion through the echolucent material 129 to the distal terminus of the catheter assembly 11. As used herein, "echolucent" refers to any material configured to achieve reduced levels of sonic scattering, sonic absorption, sonic reflection, and sonic refraction. Such materials can include room-temperature vulcanizing ("RTV") silicone, soft adhesives, hard adhesives, epoxy, urethane, plastics and/or other suitable materials. As such, when a visualization device (not shown) is advanced through the second portion 106, visualization can be achieved through the echolucent material 129 to gain information regarding anatomical conditions adjacent the surface 122, such as the vessel wall. In some embodiments the visualization lumen 108 can be a separate tube extending through the echolucent material 211, and in some embodiments the echolucent material 211 can be formed to include an elongated, tubular cavity that can serve as the visualization lumen.

Figures 6C and 6D shows an alternate embodiment of the support assembly 20. As shown in Figures 6C and 6D, the support assembly 20 may include two expandable members 22a and 22b connected to inflation lumens 116a and 116b, respectively. In this embodiment, the expandable members 22a and 22b are radially angled to either side of the trough 128. Similar to the embodiments including a single expandable member 22, the embodiment shown in Figures 6C allows a force in a direction opposing surface 122, but enables a smaller overall cross sectional profile. Other embodiments may include more than two expandable members. Additional embodiments comprise mechanical expandable members such as expandable struts, cages, meshes, braids, or the like, and be actuated via actuating members in place of an inflation lumen and/or may be self-expanding.

In some embodiments of the support assembly disclosed herein, the expandable member 22 is an inflatable structure which is sealed at both ends and connected to an inflation lumen (not visible). The inflatable structure may be one or more formed elastomeric balloons or may be one or more sections of elastomeric tubing. A formed elastomeric balloon may be blow-molded from tubing or may be tipped from a forming mandrel. Other methods of formed balloons are also possible.

Figure 6E is a cross-sectional end view of another embodiment of the support assembly 20. As shown in Figure 6E, the expandable member 22 comprises a single layer of elastomeric membrane 27. In Figure 6E, the expandable member 22 is shown in a non-expanded state. The membrane 27 is fixed with adhesive bond 29a and 29b around the perimeter of membrane 27 to the distal insert 42 and held in place by support housing 40 to create an enclosed chamber 21. The inflation lumen (not visible) enters the enclosed chamber 21 and fluidly connects the enclosed chamber 21 to a pressurized fluid source such that the pressurized fluid source can pressurize the chamber (via liquid or gas) and cause the membrane 27 to expand outwardly. In certain instances, an elastomeric membrane may be preferred over an expandable balloon or tubing. For example, the elastomeric membrane shown in Figure 6E comprises a single layer of material (as opposed to a balloon, which is two layers of material), thus allowing for a lower profile. Also, the chamber 21 formed by the membrane 27 and the distal insert 42 are not constrained by the shape of a formed balloon or inflatable tube. For example, the perimeter of the elastomeric membrane may be an irregular shape (e.g., tapered at each end, etc.).

The optional distal tip 44 of the support assembly 20 will now be described with reference to the isometric view of the support assembly 20 in Figure 7. (In Figure 7, the support assembly 20 is shown with a guidewire GW positioned through the lumen 108 of the second shaft 188 (Figures 5B and 5C).) The distal tip 44 may be a soft, flexible material that is fixed to a distal end portion of the distal insert 42 and/or support housing 40. The distal tip 44 may include a channel therethrough and an opening 44a at its distal terminus. The channel and opening 44a are configured to receive the second shaft 188, a guidewire GW (with or without the second shaft 188), and/or a visualization device (not shown) (with or without the second shaft 188.) In some embodiments, the distal tip 44 may be manufactured from a material that is softer than the more rigid support housing 40 and distal insert 42. For example, the distal tip 44 may be made of one or more of silicone rubber, polyurethane elastomer, soft Pebax^{®}, thermoplastic elastomers (such as Santoprene), and the like. The tip 44 may also be tapered in a distal direction to facilitate (i) introduction of the catheter assembly 11 into the vasculature, and (ii) advancement of the catheter assembly 11 within the vasculature, either with or without the guidewire GW received therethrough. In some embodiments, the distal tip 44 includes radiopaque materials or coatings such as barium sulfate, tungsten, and/or other suitable radiopaque substances. In a particular embodiment, the distal tip 44 includes a separate marker affixed thereto. The marker may be manufactured from radiopaque material such as tungsten or tungsten impregnated polymer, gold, platinum, platinum/iridium blend, or the like. In some embodiments, the distal tip 44 is adhered to the distal insert 42 with an adhesive bond. In another embodiment, the distal tip is insert molded to the distal insert.

### 2.3 Selected Embodiments of Handle Assemblies

Figures 8A and 8B are side and cross-sectional side views, respectively, of the handle assembly 30 shown in Figure 2A. The handle assembly 30 includes a housing 501, a first actuator 531 for axially moving the tissue penetration assembly 15, a second actuator 532 for axially moving the valve creation assembly 17, and a third actuator 533 for actuating the valve creation assembly 17 (as described in greater detail below under heading 4.0). The handle assembly 30 also comprises one or more connectors for coupling one or more lumens of the catheter assembly 11 to one or more fluid lines. For example, the handle assembly 30 shown in Figures 8A and 8B includes a first connector 534 for fluidly coupling one or more components of the catheter assembly 11 (e.g., the tissue penetration assembly 15) to a first pressurized fluid source (not shown), such as an inflation device, a syringe, a drip-bag, etc. The handle assembly 30 also includes a second connector 535 for fluidly coupling a lumen (e.g., the second lumen) in the catheter shaft to a second pressurized fluid source (not shown) for flushing the treatment site before, during, and/or after treatment. The handle assembly 30 may also include a third connector 536 for fluidly coupling a third pressurized fluid source (not shown) to the expandable member 22. The handle may include a fourth connector 539 which allows coupling of a hemostasis valve (not shown) to the lumen 108. In some embodiments, the handle assembly 30 may include more or fewer connectors and/or other connector configurations.

The first actuator 531 for translating the tissue penetration assembly 15 may be a knob which is rotationally coupled to the outside of the handle housing 501 and also mechanically coupled to the tissue penetration assembly components. In some embodiments, the tissue penetration assembly 15 is attached to a hub or hubs, which in turn are mechanically coupled via one or more couplers to an outer knob that rotates with respect to the handle housing 501. The knob may be two half-knobs 531a, 531b which, when joined, capture a coupling component protruding from a slot in the handle housing 501. The joined knob halves contain an inner helical groove so that when the knob is rotated the coupling component or components translates in a linear direction (distally or proximally). The mechanical coupling configuration allow the tissue penetration assembly 15 and attached hub or hubs to rotate with respect to the knob.

In those embodiments of the valve formation system 10 where the tissue penetration assembly 15 comprises a needle and a cover tube, the first actuator 531 is a knob which is configured to advance both the needle and the cover tube in a predetermined manner (both of which are described in greater detail below under heading 3.0 and with reference to Figures 9A-10E). For example, knob 531 may translate (proximally and distally) both the needle and the cover tube together at the same time and/or translate the needle and the cover tube separately. For example, in some embodiments, when the knob is at an initial position (e.g., before being rotated), the needle and the cover tube may be positioned such that the needle is extending distally from the cover tube (as shown in Figure 10A). Turning the knob 531 in a first direction (e.g., clockwise or counterclockwise) from the initial position causes the needle and cover tube to advance distally together at generally the same rate until the knob 531 rotates a first amount, at which point continuing to turn the knob 531 in the first direction causes the cover tube to advance relative to the needle such that the cover tube is covering the needle (e.g., the distal end of the cover tube is distal of the beveled edge of the needle, as shown in Figure 10B). In some embodiments, while the cover tube is being advanced over the needle, the needle may continue to move distally (but at a slower rate than the cover tube), and in some embodiments the needle may be held stationary while the cover tube is advanced. The first amount of rotations corresponds to a distance traveled by the needle and cover tube. After the knob 531 has been turned in the first direction a second amount beyond the first amount, continuing to turn the knob 531 causes the needle and the cover tube to be advanced together at generally the same rate. The second amount of rotations corresponds to a distance traveled by the cover tube and/or the needle. At any point during any of the foregoing processes, the knob 531 may be turned in a second direction opposite the first direction (e.g., the other of clockwise or counterclockwise) to reverse the process.

In some embodiments, a coupler internal to the housing of the handle assembly 30 is mechanically coupled to the knob 531 on the outside of the housing 501 such that rotation of the knob 531 both rotates and translates the coupler. For example, the coupler may have protruding elements that protrude through helical slots in the handle housing 501 and mate to an internal helical groove in the knob 531. Thus, as the knob is turned, the coupler both rotates and translates, as dictated by the helical slot in the handle housing 501 and the helical groove in the knob 531, respectively. The coupler in turn dictates movements of the needle and the cover tube. In some embodiments, the needle and cover tube are each attached to a proximal hub. Both hubs are configured to be constrained from rotating and also mechanically coupled to the coupler, for example, by one or more posts protruding from the hubs which mate to one or more slots in the coupler. The slot for the cover tube hub posts may simply be a circumferential slot so that, as the coupler rotates and translates via rotation of first actuator 531, the cover tube translates distally. The slot for the needle hub posts may be a cam slot that is configured so that as the coupler rotates and translates via rotation of the knob 531, the needle hub and needle first moves distally, then stops moving distally, then continues to move distally. The needle movement is dictated by the pattern of the cam slot.

The second actuator 532 which translates the valve creation assembly 17 may have a similar configuration to the first actuator 531, namely an outer knob which, when rotated by the user, translates the valve creation assembly 17 axially in a distal or proximal direction.

The third actuator 533 may be configured to actuate the valve creation assembly 17 to expand and collapse the assembly. As discussed in greater detail below under heading 4.0, in some embodiments the valve creation assembly 17 includes an outer shaft and an inner member (such as shaft 1104 in Figures 11A and 11B, shaft 1204 in Figures 12A and 12B, etc.). In such embodiments, moving the inner member proximally with respect to the outer shaft (by pulling the inner member proximally while holding the outer shaft stationary or pushing the outer shaft distally while holding the inner member stationary) expands the valve creation assembly 17, and moving the inner member distally with respect to the outer shaft collapses the valve creation assembly 17 (by pushing the inner member distally while holding the outer shaft stationary or pulling the outer shaft proximally while holding the inner member stationary). In some embodiments, the third actuator 533 is configured to move the inner member of the valve creation assembly 17 relative to the outer shaft. For example, in some embodiments the third actuator 533 is a slider that is mechanically coupled to a coupler within the housing 501, and the coupler 501 is affixed to the inner member. The slider may be configured to fit through slots on the housing 501 such that a user can translate the slider back and forth, which in turn translates the inner member with respect to the outer shaft of the valve creation assembly 17, thus expanding or collapsing the dissection arm(s) and tensioning arm(s) of the valve creation assembly 17. In some embodiments, the degree to which the valve creation assembly 17 expands is coupled to the inner member translation distance. In such embodiments, the slider may be connected to an adjustable stop which varies the travel distance of the actuator shaft and thus the expansion distance of the valve creation assembly 17 (and thus expandable size of the valve creation assembly 17). The user may manipulate the adjustable stop to the appropriate amount of expansion for a particular vessel size.

The handle assembly 30 may also include a means to connect the handle assembly 30 to a holder, for example, an instrument holder which can be clamped to a side rail of an operating table. In some embodiments, the handle assembly 30 includes a post 537 which fits into an instrument holder receptacle that is designed to hold surgical instruments and scopes, such as the Mediflex StrongArm (Mediflex Surgical Products). In this way, the handle assembly 30 can be held in the correct position without requiring the user to use one hand to hold the proximal handle. Thus, both hands of the user can be used to manipulate the proximal handle actuators, a visualization device (e.g., an IVUS catheter), one or more flush controls, or other devices or procedural manipulations as needed.

As discussed above under heading 2.1, the sidewall of the shaft 12 may comprise a single shaft tubing that is attached at its proximal portion 14 to the handle assembly 30 and the support assembly 20 at its distal portion 16. Figure 8C illustrates such an embodiment, and Figure 8D is an enlarged cross-sectional end view taken along line 8D-8D in Figure 8C. As shown in Figures 8C and 8D, the handle assembly 30 may include an actuator 543 configured to rotate the shaft 12 with respect to the handle 30. For example, in some embodiments the actuator 543 is a generally cylindrical knob 543 rotatably attached to the distal end portion of the handle housing 501. The outer knob 543 may be mechanically coupled to the proximal end portion of the shaft 12 via a gear assembly 579 such that when the outer knob 543 is rotated with respect the handle housing 501, the shaft 12 rotates. The gear assembly 579 may include an outer gear 583 fixed to the knob 543, an inner gear 581 fixed to the shaft 12, and one or more intermediate gears 582 coupling the outer gear 583 to the inner gear 581. In the embodiment shown in Figures 8C and 8D, the gear assembly 579 includes three intermediate gears (582a-582c). In some embodiments, the gear assembly 579 can include more or fewer intermediate gears (e.g., one gear, two gears, four gears, etc.). Moreover, in some embodiments, the gear assembly 579 may only include two sub-gears.

In some embodiments, the handle assembly 30 includes an actuator 544 configured to translate the shaft 12 and distal portion 20 with respect to the handle assembly 30. For the example, the actuator 544 may be a slider that is mechanically coupled to a coupler 590 which in turn is affixed to the shaft 12. The slider 544 may be configured to fit through slots 591 on the housing 501 such that a user can translate the slider 544 back and forth which in turn translates the coupler 590 and the shaft 12 back and forth. In some embodiments, the coupler 590 is configured to rotate with respect to the slider 544. For example, the coupler 590 may be a grooved ring and the slider 544 may have a feature which protrudes into the groove, or in some embodiments the coupler 590 is a ring and the slider has an inner groove which captures the ring. In some embodiments, the handle assembly 30 has two sliders to capture the coupler 590 on both sides. Such embodiments may be more mechanically stable, as the multiple sliders provide a more equal distribution of force on the coupler 590 during translation of the shaft 12. Other coupling and actuator designs which can accomplish the same functions are also possible.

In some embodiments, the actuator 544 is configured to translate the shaft 12 and distal portion 20 with respect to handle 30 and also with respect to (i.e. without also translating) valve creation assembly 15 and/or tissue penetration assembly 15 which are slideably contained within shaft 12. In these embodiments, the proximal ends of valve creation assembly 17 and/or tissue penetration assembly 15 are affixed to separate connectors within handle 30 (not shown) and which do not move when the actuator 544 is translated.

In some embodiments, the shaft 12 may comprise multiple shafts, each of which may be controlled at the handle assembly 30. Figure 8E, for example, shows one embodiment of a catheter assembly 11 where the shaft 12 comprises an inner shaft 542 and an outer shaft 541. The outer shaft 541 may be connected at its proximal portion to the housing 501 of the handle assembly 30, and the distal terminus of the outer shaft 541 may be a free end (i.e., not connected to anything). The inner shaft 542 may be rotationally and slidably disposed within at least a portion of the outer shaft 541, and connects the support assembly 20 to one or more elements in the handle assembly 30 that are configured to rotate, and translate the inner shaft 542 back and forth. Thus, the support assembly 20 may be rotated and translated while the outer surface of the catheter is fixed in the sheath and vessel. In some embodiments, the annular space between the inner shaft 542 and the outer shaft 541 is fluidly coupled to the second connector 535 (Figure 8A) on the handle assembly 30 via a flush coupler 545 and internal tubing 546. The handle assembly 30 may include elements connected to actuators 543 and 544 that are configured to rotate and translate the shaft 12. By manipulating these actuators, the user can rotate and/or translate the inner shaft 542 and attached support assembly 20 without movement of the entire handle assembly 30 and outer shaft 541. Such an embodiment facilitates desired placement and orientation of the support assembly 20 in the vessel at the target site while the handle assembly 30 is fixed via the support post and equipment holder to the OR table.

In some embodiments, the length of the outer shaft 541 with respect to the inner shaft 542 is configured such that the support assembly 20 is always exposed, through all translation positions of the inner shaft 541. In some embodiments, the length of the outer shaft 541 with respect to the inner shaft 542 is configured such that the support assembly 20 is exposed when the inner shaft 542 is translated to its distal-most position but covered by the outer shaft 541 when the inner shaft 542 is translated to its proximal-most positon.

In some embodiments, all of the handle actuators (including those that actuate the tissue penetration assembly 15 and valve creation assembly 17) may be configured to allow for rotation of the components.

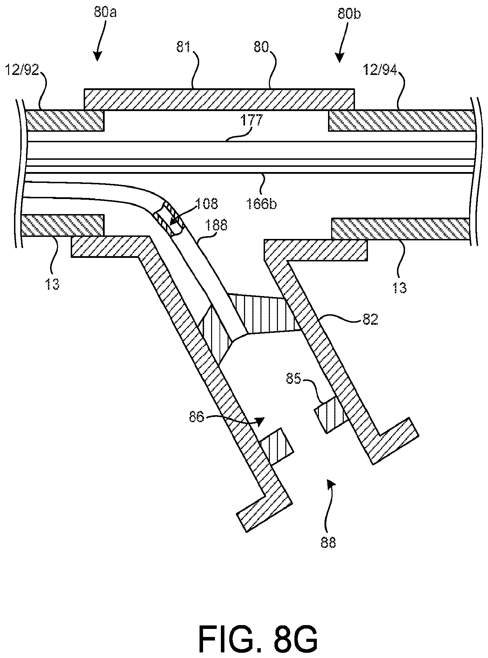

In some embodiments, the shaft 12 may include an extension at its proximal portion. For example, Figure 8F shows a shaft 12 including an extension 80 (e.g., a Y-arm component) positioned at its proximal portion 14 distal of the handle assembly 30, and Figure 8G is a cross-sectional view of the extension 80. Referring to Figures 8F and 8G together, the extension 80 may be integral to the shaft 12 or may be a separate component configured to permanently or detachably couple to a proximal portion of mid-section of the shaft 12. The extension 80 may include a main body 81 and a side arm 82 defining a lumen 86 extending to an opening 88. The side arm 82 may be configured to receive another intravascular device therethrough (a guidewire, an imaging catheter, etc.) In some embodiments, the extension 80 includes a valve 85, such as a hemostasis valve, positioned along the side arm 82. The valve 85 may be integral to the side arm 82 and/or extension 80 or may be a separate component configured to permanently or detachably couple to the side arm 82 and/or extension 80. The extension 80 may be made of one or more polymers (e.g., a plastic) and/or one or more other suitable materials. The valve 85 may be a passive valve such as a septum valve or a rotating hemostasis valve such as a Tuohy Borst valve.

The extension 80 may be configured such that, when the extension 80 is coupled to the shaft 12, the lumen 86 is in fluid communication with the second lumen 108 such that the second lumen 108 extends proximally from the opening 88 at the end of side arm 82 to the opening 109 (see Figure 5A) at the distal tip 44 of the catheter 11. For example, the second lumen 108 may be directed towards the side arm 82 and bonded in place to create a continuous path out through the lumen the lumen 86 and valve 85. As such, an intravascular device may be inserted into the second lumen 108 at a proximal portion of shaft 12 through valve 85 and side-arm 82, rather than be inserted through the entirety of shaft 12 as well as then length of handle assembly 30 (as shown in Figure 2A). This feature allows a shorter intravascular device (e.g., a shorter guidewire or shorter imaging catheter) to be used with the valve formation system 11.

In those embodiments where the extension 80 is a separate component from the shaft 12 (such as that shown in Figures 8F and 8G), the shaft 12 may be split into a distal segment 92 and a proximal segment 94, with a distal end 80a of the extension 80 coupled to the proximal end of distal shaft segment 92, and a proximal end 80b of the extension 80 coupled to the distal end pf proximal shaft segment 94. The second lumen 108 is directed towards the side-arm and bonded in place to create a continuous path out the valve 85. The remaining lumens in shaft 12 (first lumen 107 and third and fourth lumens 116a and 116b) continue from the distal segment of shaft 12, through the main body 81 of the Y-arm 80 and into the proximal segment 94 of shaft 12 to enter handle assembly 30. The distance of the Y-arm 80 from the handle 30 is such that the shaft 12 may be translated into handle 30 as required without interference from the Y-arm. In other words, the distance is the same or greater than the shaft translation distance.

### 3.0 Selected Embodiments of Tissue Penetration Assemblies and Methods of Use

Figure 9A is a side cross-sectional view of a tissue penetration assembly 15 configured in accordance with the technology. The tissue penetration assembly 15 is configured to puncture the vessel wall and be advanced within the vessel wall while ejecting fluid to separate vessel wall tissue, thereby forming a space S within the vessel wall (see Figures 3A-3D). As used herein, the term "puncture" refers to an action that gains entry to an interior portion of the vessel wall without crossing through the entire thickness of the vessel wall.

Figure 9B is a cross-sectional end view taken along lines 9B-9B in Figure 9A. Referring to Figures 9A and 9B together, the tissue penetration assembly 15 comprises a tissue penetrating element 110 formed of a tubular wall 111 having an inner surface that defines a lumen 112. The tissue penetrating element 110 may have a beveled or slanted distal face 160 and an exit port 124 positioned along the distal face 160. The lumen 112 is configured to receive a fluid therethrough and can have a proximal portion (not shown) coupled via a proximal adaptor (such as a Luer hub) to a pressurized fluid source (e.g., a syringe, a pump, an inflation device, a mechanical fluid pressurizer, etc.). The lumen 112 extends distally from the proximal portion to the exit port 124, and the tissue penetrating element 110 is configured to eject the fluid through the exit port 124. The wall 111 can extend distally from the proximal portion of the tissue penetrating element 110 to the distal face 160.

The distal face 160 can have a distal-most puncturing edge 126 (shared with a distal terminus of the wall 111) configured to puncture a vessel wall, and a proximal-most edge 128. As shown in Figure 9A, the distal-most edge 126 and the proximal-most edge 128 can be positioned opposite one another about a circumference of the tissue penetrating element 110. In someIn some embodiments, the distal-most edge 126 and the proximal-most edge 128 can have other arrangements. In some embodiments, the distal face 160 is beveled to create a needle point. In a particular embodiment, the needle point is a lancet point geometry with two angled bevels ground into the distal face to create a cutting point and two cutting edges. In some embodiments, the tissue penetrating element 110 can include a plug 120 positioned within the lumen 112 along all or a portion of its length and blocking a portion of the exit port 124. The plug has the effect of narrowing the exit port at the distal tip so that the flow pattern is improved for hydrodissecting tissue layers without creating a high flow resistance along the entire length of the tissue penetrating element 110. In certain embodiments, the plug 120 narrows the exit port 124 such that the exit port 124 is concentric to the tissue penetrating element 110. In the embodiment shown in Figures 9A and 9B, the plug 120 is configured to off-set the longitudinal axis P of the exit port from a longitudinal axis A of the tissue penetrating element 110. As such, the entire exit port 124 is positioned nearer the distal puncturing edge 126 of the tissue penetrating element 110. In a particular embodiment, at least a portion of the distal face 160 can be formed from the distal-most surface of the plug 120.

As shown in Figure 9B, the plug 120 may be configured to provide an exit port 124 with a generally circular cross section. In an alternative embodiment shown in Figure 9C, the plug 120 is configured to form an exit port 124 with a generally D-shape.

The plug 120 can be a separate component fixed to the wall 111 via adhesive, soldering, welding, etc. In some embodiments, the plug 120 can be integral with the wall 111. For example, during manufacturing, the wall 111 can be extruded to include the plug 120. In some embodiments, the plug 120 can have other suitable shapes, sizes, and/or configurations. For example, in some embodiments, the plug 120 can have a generally constant thickness along its length and can extend along all or a portion of the tissue penetrating element 110.

In some embodiments, the tissue penetrating element 110 has a diameter that can puncture the tissue layer but is small enough so that inadvertent puncture of the vessel wall will not cause a clinically significant perforation. In a particular embodiment, the tissue penetrating element 110 has a hypodermic needle gauge size of between 22 and 26 and a wall thickness of between about 0.002 inches and about 0.004 inches. In certain embodiments, the needle gauge is 25 with an outer diameter of about 0.020 inches and a wall thickness of about 0.002 inches, with an inner lumen diameter of about 0.016 inches. In a particular embodiment, the exit port 124 may have a height or diameter (depending on if it is D-shaped or circular) between about 0.004 inches and about 0.010 inches. In a particular embodiment, the exit port 124 has a height or diameter of about 0.008 inches. In this embodiment, the offset of the exit port axis P with respect to the longitudinal axis A is about 0.004 inches. In some embodiments, the tissue penetrating element 110 may have other suitable offset amounts and exit port sizes and shapes.

In some embodiments, the tissue penetration assembly 15 additionally comprises a cover tube 140 for covering the tissue penetrating element 110 after the tissue penetrating element 110 has entered the vessel wall through an opening. As shown in Figures 10A and 10B, the cover tube 140 is slidably disposed around the penetrating element 110. During tissue puncture, the cover tube 140 is positioned to expose the tip of the penetrating element 110, as shown in Figure 10A. After puncture and during advancement of the tissue penetration assembly 15 when creating space S, the cover tube 140 may advance distally with respect to the penetrating element 110 to cover the penetrating element tip, as shown in Figure 10B. This allows the tissue penetration assembly 15 to advance and create a space within the interior portion of the vessel wall without risk of perforating the vessel wall outward (through the vessel wall to a position outside of the vessel) or inward (through the vessel wall into the true lumen). In the present embodiment, the tissue penetrating element 110 and the cover tube 140 can be connected to components in the handle assembly 30 and configured such that movement of the first actuator 531 (i) advances the tissue penetration assembly 15 with the penetrating element 110 exposed, (ii) after puncture, advances just the cover tube 140 over the penetrating element 110 until the penetrating element 110 is covered, and (iii) advances the entire tissue penetration assembly 15 until the desired length of travel is achieved to create a space in the vessel wall. In some embodiments, the tissue penetration assembly 15 and catheter assembly 11 includes features which radially align the beveled surface 160 of the tissue penetrating element 110 with the surface 122 of the support assembly 20. For example the handle assembly 30 can fix the tissue penetration assembly 15 radially with respect to the shaft 12 and support assembly 20 so that the beveled surface 160 is oriented with respect to the surface 122 of support assembly 20 such that the distal-most edge 126 is furthest from the surface 122 and the proximal most edge 128 is closest to surface 122. This alignment enables access to a precise and repeatable thickness of vessel wall layer.

As illustrated in Figures 10C-E, hydrodissection of the vessel wall VW cannot occur until the entire exit port 124 of the tissue penetrating element 110 has been advanced into the vessel wall VW. This is because the pressurized fluid moving through the lumen 112 will take the path of least resistance. As such, when only a portion of the exit port 124 is positioned within the vessel wall, the ejected fluid encounters a large resistance at the portion of the exit port in contact with the vessel wall tissue, and only a very small resistance at the portion of the exit port fluidly coupled to the vessel lumen (as shown in Figure 10C). Once the tissue penetrating element 110 enters further such that the entire exit port 124 is in the tissue layer, as shown in Figure 10D, the fluid flow entirely into the tissue layers and begins separating the layers (e.g., hydrodissection) without the entire diameter of the tissue penetrating element 110 being advanced into the vessel wall VW. This configuration enables a very thin layer of vessel to be accessed and subsequently hydrodissected. For example, in some embodiments, the puncture depth can be between about 1/6 to about 1/2 of the vessel wall thickness (assuming an average vessel wall thickness of about 1 mm), or between about 0.008 inches to about 0.020 inches. Once the tissue penetrating element 110 is well within the tissue layers and advancing to form space S, the cover tube 140 is advanced over the tissue penetrating element 110 so that hydrodissection of space S can continue without risk of perforation of the vessel wall layers by tissue penetrating element 110 (as shown in Figure 10E).

In some embodiments, the tissue penetration assembly 15 may be used as a guide to advance a valve creation assembly 17, as described further below. In this embodiment, the cover tube 140 also serves to provide a transition between the outer diameter of the tissue penetrating element 110 and the inner diameter of the valve creation assembly 17. In the embodiment shown in Figures 10A and 10B, the cover tube 140 has a tapered distal end portion 141 that creates a smooth transition in this gap. The taper may be formed by beveling the cover tube material. The taper may alternatively be formed by inserting a second smaller diameter tubing into the cover tube 140 and applying a tapered bead of adhesive around the transition from one tubing to the other to create the taper or heating the smaller diameter tubing to heat from a taper. In another embodiment, the cover tube 140 may step up from one diameter to a larger diameter to provide some of the transition in this gap. It will be appreciated that any of the tissue penetration assembly embodiments described herein can be used regardless of the overall shape and/or configuration of the tissue penetration assembly 15 or valve creation assembly 17.

### 4.0 Selected Embodiments of Valve Creation Assemblies and Methods of Use

Embodiments of the valve creation assembly 17 are now described, with reference to the anatomical structures in Figures 3A-3F. Valve creation assemblies according to the present technology generally comprise a dissection device and a cutting device. The dissection device can enlarge the space S created by the tissue penetration assembly 15 in order to form a dissection pocket DP, while the cutting device can cut tissue at the opening O to form mouth M and transform the dissection pocket DP into a valve leaflet L. In some embodiments, the dissection device and cutting device are the same device configured to perform both the dissection function and the cutting function. In another embodiment, the dissection device and the cutting device are two separate devices that are exchanged one device for the other to perform the dissection and cutting functions. In yet another embodiment, the dissection device and the cutting device can be slidably coupled such that they can be delivered at the same time without requiring an exchange of one for the other.

Figures 11A-11D illustrate an embodiment of a valve creation assembly 1117, in accordance with the present invention, in which the dissection device and cutting device are the same device, shown in a low-profile state and a deployed state, respectively. The valve creation assembly 1117 can include an outer elongated shaft 1102 and an inner elongated shaft 1104 slidably disposed within a lumen of the outer shaft 1102. The outer shaft 1102 can have distal portion 1106 that includes dissection arms 1108, distal end region 1110, and tension arm 1112. In the embodiment shown in Figures 11A and 11B, one or more regions of the shaft 1102 have been removed along the distal portion 1106 to form the dissection arms 1108 and tension arm 1112. In some embodiments, the dissection arms 1108 and tension arm 1112 can be separate components coupled to the shaft 1102. The distal end of inner shaft 1104 can be fixed to or forcibly coupled to the distal end region 1110 of the outer shaft 1102. As such, actuation of the inner shaft 1104 with respect to the outer shaft 1102 can force the dissection arms 1108 and tension arm 1112 into the deployed state shown in Figure 11B. Specifically, proximal movement of the inner shaft 1104 with respect to the outer shaft 1102 (as indicated by arrow A in Figure 11B) pulls the distal portions of the dissection arms 1108 and tension arm 1112 proximally and forces the dissection arms 1108 and tension arm 1112 to bend outwardly away from the longitudinal axis of shaft 1102, as shown in Figure 11B. When the dissection arms 1108 are positioned within a space S in the vessel wall W, expansion of the dissection arms 1108 serves to separate one or more tissue layers and to thereby expand the space S to form dissection pocket DP.

In some embodiments, the valve formation assemblies described herein have a central lumen 1187, as seen in Figure 11A and 11B. The interior shaft 1104 defines the central lumen 1187, and lumen 1187 enables the valve creation assembly 1117 to be slidably disposed over a tissue penetration assembly 15 in order to be positioned in the tissue space S to form dissection pocket DP. In some embodiments, the tissue penetration assembly is removed prior to insertion of the of the valve creation assembly.

The dissection arms 1108 can include one or more segments 1109 (referred to individually as first and second segments 1109a and 1109b) and one or more joints 1114 (referred to individually as first-third joints 1114a-c). The joints 1114 can be positioned along the dissection arms 1108 between successive segments 1109 and/or at portions of the arms 1108 that meet the shaft 1102 (e.g., the proximal and distal end portions of arms 1108). The joints 1114 can be portions of the dissection arms 1108 and/or shaft 1102 configured to preferentially flex relative to segments 1109 and/or the shaft 1102. In some embodiments the joints 1114 can be formed by opposing recesses or a thinned section at a desired position along the arm 1108 (e.g., a living hinge). In some embodiments, one or more of the joints 1114 can be one or more small pins, elastic polymeric elements, mechanical hinges and/or other devices that enable one segment 1109 to pivot or bend relative to another.

In the embodiment shown in Figures 11A and 11B, each of the dissection arms 1108 includes a distal segment 1109a, a proximal segment 1109b, a distal joint 1114a at the distal end of distal segment 1109a, a proximal joint 1114c at the proximal end of proximal segment 1109b, and an intermediate joint 1114b positioned along the length of the respective arm 1108 between the proximal and distal joints 1114a and 1114c and connecting the segments 1109. In response to longitudinal stresses caused by actuation of the interior shaft 1104, the dissection arms 1108 deform into a predetermined shape based on the configuration and/or relative positions of the joints 1114. For example, in the embodiment illustrated in Figures 11A and 11B, each of the dissection arms 1108, when deployed, includes generally linear distal segment 1109a and generally linear proximal segment 1109b that, taken together, enclose a kite-like shape. When fully deployed, the distal segments 1109a can be generally parallel so that the arms 1108, taken together, enclose a generally triangular shape. In some embodiments, one or both of the segments 1109 has a generally curved shape such that the dissection arms 1108, taken together, form a rounded triangular or "shield-like" shape. In some embodiments, the number of segments, the length of each segment, the angle between segments, and/or the shape of each segment (e.g., linear, curved, etc.) can be varied along a single dissection arm and/or amongst a plurality of dissection arms to achieve a desired dissection pocket DP and/or leaflet L shape. Moreover, the dissection arms 1108 can have any suitable size and/or shape based on a desired bending stiffness, angle, and radius of curvature. Additionally, the deployed shape of the dissection arms 1108 and/or the amount of tissue separated by the dissection arms 1108 may be adjusted by varying the actuation of shaft 1104 to account for valve formation in different vessel diameters.

The tension arm 1112 can have a generally similar structure as dissection arms 1108. For example, tension arm 1112 can include one or more segments 1113 (referred to individually as first and second segments 1113a and 1113b) and one or more joints 1116 (referred to individually as first-third joints 1116a-c). The joints 1116 can be positioned along the tension arm between successive segments 1113 and/or at portions of the arm 1112 that meets the shaft 1102 (e.g., the proximal and distal end portions of arm 1112). The joints 1116 can be portions of the tension arm 1112 and/or shaft 1102 configured to preferentially flex relative to segments 1113 and/or the shaft 1102. In some embodiments the joints 1116 can be formed by opposing recesses or a thinned section at a desired position along the arm 1112 (e.g., a living hinge). In some embodiments, one or more of the joints 1116 can be one or more small pins, elastic polymeric elements, mechanical hinges, and/or other devices that enable one segment 1113 to pivot or bend relative to another.

In the embodiment shown in Figures 11A and 11B, tension arm 1112 is oriented in an angular direction 90 degrees from the dissection arms 1108. Figure 11A is an isometric view of the valve creation assembly 1117 in which the tension arm 1112 is in a low-profile state. Figure 11B shows the tension arm 1112 in a deployed state that is at a generally opposite angle with respect to shaft 1102 compared to the low-profile illustration of the arm 1112 in Figure 11A. Tension arm 1112 includes distal segment 1113a, proximal segment 1113b, distal joint 1116b at the distal end of distal segment 1113a, proximal joint 1116c at the proximal end of proximal segment 1113b, and intermediate joint 1116b positioned along the length of the tension arm 1112 between the proximal and distal joints 1116a and 1116c and connecting the segments 1113. Similar to the dissection arms, in response to longitudinal stresses caused by actuation of the interior shaft 1104, the tension arm deforms into a predetermined shape based on the configuration and/or relative positions of the joints 1116. For example, in the illustrated embodiment, tension arm 1112 includes segments 1113 that both have a generally linear shape. In some embodiments, one or both of segments 1113 can have a generally curved shape. More generally, the number of segments 1113, the length of each segment 1113, the angle between segments 1113, and/or the shape of each segment 1113 (e.g., linear, curved, etc.) can be varied along a single tension arm and/or amongst a plurality of tension arms. Moreover, the tension arm 1112 can have any suitable size and/or shape for creating more volume during expansion of the space S and/or for adding tension to the tissue to facilitate the cutting step. For example, the tension arm 1112 may have only two joints at the distal and proximal ends of the arm, and when actuated form one continuous arc rather than a segmented arm.

Other variations of tension arms and dissection arms can exist. For example, other embodiments can have variations on dissection and tension arm joint locations, dissection and tension arm lengths, and cutting element location and lengths. In any of these variations, the valve formation assembly may be configured to open a different amount during the valve formation step, to take into account different vessel sizes.

In some embodiments wherein the valve creation assembly comprises both a dissection device and a cutting device, cutting elements 1185 are disposed on the dissection arms 1108. Each of the cutting elements 1185 can have a sharp edge configured to cut vessel wall tissue. The cutting elements 1185 may be a separate component coupled or attached to one or both of dissection arms 1108, or may be integrally formed with the dissection arms 1108. The cutting elements are generally positioned along the dissection arms 1108 to cut vessel wall tissue at the opening O in vessel wall W. Specifically, the cutting elements 1185 can cut sideways the opening O to widen the opening in order to a create mouth M for the dissection pocket DP.

In the embodiment illustrated in Figures 11A and 11B, valve creation assembly 1117 includes cutting elements 1185 attached to the proximal segments 1109b of the dissection arms 1108. When the dissection arms 1108 are expanded, the cutting elements 1185 are angled towards the proximal end of the valve creation assembly 1117. The cutting step may occur by expanding the dissection arms 1108 and the tension arm 1112, and then pulling the valve creation assembly in the proximal direction when the cutting elements 1185 are positioned at or near the opening O in the vessel wall W. In this embodiment, the proximal segments 1109b of the dissection arms 1108 are longer than the distal segments 1109a, allowing for a more acute angle which may ease the cutting step. In some embodiments, the cutting step can occur by simply expanding the dissection arms 1108 when the cutting elements 1185 are positioned within the opening O without translating the valve creation assembly 1117. Moreover, one or more tension arms 1112 can be in a deployed state to provide tension to the tissue to ease the cutting step.

Figures 11C and 11D show one method of attaching cutting elements 1185 to dissection arms 1108. Figure 11C shows just the distal portion 1106 of outer shaft 1102, without inner shaft 1104. The cutting elements 1185 are latched onto the dissection arms 1108 by means of latch components 1190. As shown more clearly in exploded view in Figure 11D, the cutting elements 1185 have two or more tabs 1186. Each tab has a slot 1188. The tabs fit through slots 1195 in dissection arms 1108. Once through, a latch component 1190 is configured to fit through slots 1188, thus locking the cutting element 1185 to dissection arm 1108. Other means are possible to attach cutting elements 1185 to dissection arms 1108, for example the cutting elements may be mechanically attached with alternate locking means, welded, soldered, or bonded to dissection arms.

Figures 12A and 12B illustrate another embodiment of a valve creation assembly 1217 in which the dissection device and cutting device are the same device, shown in a low-profile state and a deployed state, respectively. Valve creation assembly 1217 includes generally similar features as valve creation assembly 1117 described above with reference to Figures 11A and 11B. For example, valve creation assembly 1217 includes dissection arms 1208, each arm having distal segment 1209a, proximal segment 1209b, distal joint 1214a at the distal end of distal segment 1209a, proximal joint 1214c at the proximal end of proximal segment 1209b, and an intermediate joint 1214b positioned along the length of the respective arm 1208 between the proximal and distal joints 1214a and 1214c and connecting the segments 1209.

In contrast to the embodiment illustrated in Figures 11A and 11B, cutting elements 1285 are disposed on the distal segments 1209a of the dissection arms 1208, and distal segments 1209a are longer than proximal segments 1209b. Therefore, in response to longitudinal stresses caused by actuation of the interior shaft 1204, the dissection arms 1208 deform into a generally triangular or kite-like shape in which the cutting elements 1285 are angled towards the distal end of the valve creation assembly 1217. The cutting step occurs after the valve formation assembly 1217 is pulled back to a position within the opening O (e.g., half-way in and half-way out of the opening). Expansion of the dissection arms 1208 causes the cutting elements 1285 to engage vessel wall tissue at the opening O and to cut and widen the opening to create a mouth M to the dissection pocket DP. One or more tension arms 1212 may be in a deployed state to provide tension to the tissue to ease the cutting step. Alternately, the cutting step may occur by expanding the dissection arms 1208 and then pushing the valve creation assembly distally when the cutting elements 1285 are positioned at or near the opening O in the vessel wall W.

An exemplary method of valve formation using valve creation assembly 1117 or 1217 will now be described. To begin, the valve creation assembly 1117 can first be intravascularly positioned adjacent a treatment site within a blood vessel V (e.g., a vein). To do so, the valve creation assembly is inserted in a low-profile state through the device lumen 107 of catheter assembly 11. The valve creation assembly 1117 is then positioned in the space S within the vessel wall W in a low-profile state. Specifically, the assembly is advanced distally through exit port 52 on the slanted surface 54 of the support assembly 20 and through the opening O in an interior surface IS of the vessel wall W. While positioned within the wall W, the valve creation assembly 1117 is then actuated to bend the dissection arms 1108 outwardly away from the longitudinal axis of the shaft 1102. As the dissection arms 1108 move outwardly, the dissection arms 1108 push against the tissue at the inner periphery PE of the space S, thereby separating the tissue at the periphery to enlarge the space S within the vessel wall W. The amount of actuation may be varied according to the desired size of dissection pocket DP (and ultimate size of the formed valve leaflet). For example, the actuation of the assembly 1117 can be controlled by the user to be opened 20%, 30%, 40%, 50%, 60%, or 70% larger than the diameter of the vessel. The over-expansion above the diameter of the vessel creates a desired pocket size DP to form an optimal valve leaflet L. Additionally, the actuation may occur step wise, for example first opened partially to initiate a dissection plane P, and then opened to the full desired size. In some embodiments, the valve creation assembly 1117 is then collapsed into a low-profile state, pulled back proximally within the space S a discreet amount, and then actuated again. A series of actuations can be repeated along the length of space S until a desired dissection pocket DP configuration is achieved. For example, in one embodiment, the valve creation assembly 1117 is actuated, collapsed, and pulled back 3-8 times to create the desired dissection pocket geometry. The number of actuations depends on the length of insertion of the valve formation assembly in space S, the amount of pull back between actuations, and the location of the opening.

At a point in the valve formation steps, the valve formation assembly 1117 is situated partially in and partially out of dissection pocket DP such that the cutting elements 1185 are at the level of the opening O and may cut the opening to create a mouth M. In the version of valve formation using either valve creation assembly 1117 or 1217, the cutting step may be performed by positioning the dissection arms 1108/1208 within the opening O and expanding the dissection arms 1108/1208 such that the cutting elements 1185/1285 engage vessel wall tissue at the opening O. Alternatively, using valve creation assembly 1117, this step can be performed by keeping the valve creation assembly 17 expanded and pulling back proximally the assembly to create a mouth cut M. In this step, the cutting elements 1185 can create the mouth cut M when the assembly is pulled proximally because the cutting elements 1185 are angled toward the proximal end of shaft 1102 when the dissection arms 1108 are expanded. Similarly, in the version of valve formation using valve creation assembly 1217, the cutting step can be performed by locating the assembly 1217 partially within the opening O and pushing the assembly distally to create the mouth M.

In a variation of this method, the valve creation assembly 1117 may be re-advanced into the pocket and re-actuated and translated to further dissect the pocket. In this embodiment, the tissue penetration element may remain in the pocket during valve creation, to guide the valve creation assembly 1117 back into the pocket. In one example, the valve creation assembly 1117 may be partially and/or fully opened, then closed and translated back a discreet amount for multiple dissection steps until a certain translation distance has been achieved Then, the valve creation assembly 1117 is re-advanced back into the pocket, fully opened and translated back while open for a final dissection step until the pocket has been fully formed and the mouth cut has been created. In another example, the valve creation assembly dissects the pocket to a slightly under-expanded size, then is re-advanced to dissect the pocket at a fully expanded size. Other dissection step configurations are also possible.

In some embodiments of the present technology, the valve creation assembly can include a separate dissection device. Figures 13A and 13B are isometric views of a separate mechanical dissection device 1300 for use in valve creation assembly 17, shown in a low-profile state and a deployed state, respectively. The dissection device 1300 is configured to enlarge a space within the sub-intimal region of a blood vessel and transform the space into a dissection pocket having a particular geometry. As shown in Figures 13A and 13B, the dissection device 1300 includes a first elongated shaft 1302, dissection arms 1308 at the distal region of the shaft 1302, and a second elongated shaft 1304 slidably disposed within a lumen of the first elongated shaft 1302. The dissection arms 1308 can include generally similar features to the dissection arms 1108 and 1208 described above with reference to Figures 11 and 12. For example, the dissection arms 1308 can include distal segment 1309a, proximal segment 1309b, distal joint 1314a, intermediate joint 1314b, and proximal joint 1314c. Actuation of the interior shaft 1304 causes the dissection arms 1308 to expand into a predetermined shape that depends on the amount of actuation, the number of segments 1309, the length of each segment 1309, the angle between segments 1309, and/or the shape of each segment 1309 (e.g., linear, curved, etc.). For example, in the illustrated embodiment, each of the dissection arms 1308, when fully deployed, includes a generally curved distal segment 1309a and a generally linear proximal segment 1309b that, taken together, enclose a rounded triangular or "shield-like" shape. In some embodiment, dissection device 1300 also includes one or more tension arms. In some embodiments, the dissection element 1300 is another mechanically expanding device such as a braided structure or a slotted tube structure which can be shorted in length to expand in diameter.

In some embodiments of the present technology, the valve creation assembly can include a separate cutting device. Figure 14 shows an exemplary cutting device 1460 for us in a valve creation assembly, shown in a deployed state. The cutting device 1460 can include an elongated shaft 1464, two cutting elements 1421 coupled to a distal region of the shaft 1464, and an actuator 1462 extending through at least a portion of the shaft 1464. Each of the cutting elements 1421 can have a sharp edge 1425 configured to cut vessel wall tissue. The elongated shaft 1464 can further include an aperture 1467 at its distal region, and can terminate distally at a rounded or atraumatic distal tip portion 1417. The aperture 1467 can have lateral openings 1424 (only one visible in Figure 14). The cutting elements 1421 can be rotatably coupled to the shaft 1464 by a first linkage 1469 and configured to pivot or rotate about the first linkage 1469 between a low-profile or collapsed state (not shown) and a deployed state in which the cutting elements 1421 extend outwardly away from a longitudinal axis of the shaft 1464. In the embodiment shown in Figure 14, the first linkage 1469 is a pin that extends from the elongated shaft 1464 across the aperture 1467 through a slot in each of the cutting elements 1421. In some embodiments, the cutting elements 1421 can be coupled to the shaft 1464 by other suitable mechanical linkages. The cutting elements 1421 can be coupled to a distal portion of the actuator 1462 by a second linkage (not visible in Figure 14). In the embodiment shown in Figure 14, the second linkage is a pin that extends from the actuator 1462 through a thickness of each of the cutting elements 1421. In some embodiments, the cutting elements 1421 can be coupled to the actuator 1462 by other suitable mechanical linkages. The first linkage 1469 can be fixed relative to the elongated shaft 1464, while the second pin can move axially relative to the shaft 1464.

In the low-profile state (not shown), the cutting elements 1421 can be generally aligned with the elongated shaft 1464 such that the majority of each cutting element 1421 lies within the lateral boundaries of the elongated shaft 1464. In some embodiments, each of the cutting elements 1421 in their entireties lies within the lateral boundaries of the elongated shaft 1464. To deploy the cutting device 1460, the actuator 1462 can be pushed distally (e.g., from the proximal portion), thereby urging the cutting elements 1421 in a distal direction. As the cutting elements 1421 are urged distally, the individual slots slide along the first linkage 1469, thereby forcing the cutting elements 1421 to rotate based on the shape of each slot. As the cutting elements 1421 rotate, they extend laterally through the openings 1424 in the elongated shaft 1464. In some embodiments, the cutting device 1460 can be configured such that proximal movement of the actuator 1462 can deploy the cutting elements 1421.

The cutting elements 1421 can extend from the shaft 1464 in a distal direction such that the cutting elements 1421 are angled with respect to the longitudinal axis of the shaft 1464. In the embodiment shown in Figure 14, sharp edges 1425 of the cutting elements 1421 face distally when the cutting elements 1421 are in a deployed state. In some embodiments, the cutting elements 1421 can extend from the shaft 1464 in a proximal direction and/or the sharp edges 1425 can face proximally when the cutting elements 1421 are in a deployed state.

In some embodiments of the valve creation assembly, separate dissection and cutting devices can be slidably coupled such that they may be delivered at the same time without requiring an exchange of one for another. For example, Figures 15A and 15B show one embodiment of a valve creation assembly 1517 that is configured to receive a separate cutting device 1560 (such as, e.g., the cutting device 1460 illustrated in Figure 14). Figures 15A and 15B show the valve creation assembly 1517 in proximal and distal deployed states, respectively. The valve creation assembly 1517 includes an elongated shaft 1502 and a pull member 1562 slidably disposed within the shaft 1502. The pull member 1562 can be a hollow tubular structure having a distal end region 1550, and the cutting device 1560 can be configured to be slidably disposed within a lumen of the pull member 1562. The elongated shaft 1502 can have a distal portion 1506, dissection arms 1508 at the distal portion 1506, and a distal end region 1510 coupled to the distal end region 1550 of the pull member 1562.

In the embodiment shown in Figures 15A and 15B, the dissection device has generally similar features as described above. For example, one or more regions of the shaft 1502 have been removed at the distal portion 1506 to form the dissection arms 1508. As such, the dissection arms 1508 can be continuous and/or integral with the shaft 1502. In some embodiments, dissection arms 1508 can be separate components coupled to the distal portion 1506 of the shaft 1502. The arms 1508 can include three joints 1514 (referred to individually as first-third joints 1514a-1514c) and two segments 1509 (individually labeled first and second segments 1509a, 1509b). The dissection arms 1508 can be actuated to deform into a predetermined shape based on the configuration and/or relative positions of the joints 1514 and segments 1509.

The elongated shaft 1502 can further include two slots 1534 along at least a portion of its length. (Only one slot 1534 is visible in Figures 15A and 15B.) In some embodiments, the slots 1534 can be positioned at circumferentially opposing portions of the shaft 1502. In some embodiments, the slots 1534 can have other suitable spacing about the circumference of the shaft 1502. In the embodiment shown in Figures 15A and 15B, each of the slots 1534 extend distally along the shaft 1502 to the second or proximal-most segment 1509b of a respective arm 1508. In some embodiments, the slots 1534 may extend to other locations along the shaft 1502 and/or corresponding arm 1508, such as a location distal to the second or proximal-most segment 1509b.

The cutting device 1560 (such as, e.g., cutting device 1460 in Figure 14) can be positioned within the shaft 1502 such that the cutting elements 1521 are circumferentially aligned with the slots 1534 along the shaft 1502. Accordingly, when the cutting elements 1521 are in the deployed state, the cutting elements 1521 extend outwardly through the slots 1534 away from the longitudinal axis of the shaft 1502. The cutting elements 1521 can extend from the shaft 1502 in a proximal direction such that the cutting elements 1321 are angled with respect to the longitudinal axis of the shaft 1502. In alternative embodiments, the cutting elements 1521 can extend from the shaft 1502 in a distal direction (such as, e.g., the embodiment shown in Figure 14). In the embodiment shown in Figures 15A and 15B, the sharp edges 1525 of the cutting elements 1521 face distally when the cutting elements 1521 are in a deployed state. In some embodiments, the sharp edges 1525 can face proximally when the cutting elements 1521 are in a deployed state.

In use, the cutting device 1560 is actuated outside (proximal to) the pocket and slid distally to cut the opening O and create a mouth M to the dissection pocket DP. Alternately, the cutting device 1560 could be expanded inside the pocket and pulled proximally to create the cut at the opening O, if the cutting edge 1521 of the blades 1525 were facing the other way (e.g., as in Figure 14). The dissection arms 1508 can be expanded or not expanded during the cutting step, but preferentially would be in the expanded state to provide tension to the vessel layer and facilitate cutting of the tissue. In a variation of this embodiment, the valve creation assembly 1517 includes one or more tension arms (not shown).

One method of using the valve creation assembly 1517 will now be described. The distal portion 1506 is first advanced through an opening O in a vessel wall W and positioned within an access space S. The pull member 1562 can be pulled proximally relative to the elongated shaft 1502 and the cutting device shaft 1564 (not shown) to bend the dissection arms 1508 away from the longitudinal axis of the shaft 1502 to form a dissection pocket DP. The cutting device 1560 can then be advanced and/or otherwise positioned within or near the dissection pocket DP in a low-profile state (not shown). The cutting device 1560 is then actuated to pivot the cutting elements 1521 into the deployed state, away from the longitudinal axis of the pull member 1562. Depending on the configuration of the cutting elements 1521, the cutting elements 1521 can deploy fully or substantially within an interior region defined by the deployed arms 1508 (e.g., within the dissection pocket DP), or can be deployed outside of the dissection pocket DP as illustrated in Figure 15A. The shaft 1564 of the cutting device 1560 can then be pulled proximally or pushed distally relative to the shaft 1502 to pull or push the cutting elements 1521 through the slots 1534 along the second or proximal-most segments 1509b of the dissection arms 1508. In the embodiment illustrated in Figures 15A and 15B, the cutting elements are pushed distally through the slots 1534. As the cutting elements 1521 pass through the slots 1534 in the respective arms 1508, all or a portion of the length of each sharp edge 1525 engages and cuts tissue adjacent the opening O in the vessel wall W to form a mouth M. In some embodiments, the degree of rotation of the cutting elements 1521 and/or the angle at which the cutting elements 1521 extend from the shaft 1564 can be adjusted depending on the length or shape of the mouth M desired. Likewise, the distance the shaft 1564 is pulled proximally or pushed distally can also be varied to achieve a desired length or shape of the mouth M.

Figures 16A-16D show a separate dissection device 1600 for use in a valve creation assembly not part of the present invention and that includes separate dissection and cutting devices. The dissection device 1600 includes an inflatable balloon 1626. Figures 16A and 16B show a front view and side view, respectively, of the dissection device 1600 inserted into the space S in vessel W with the balloon 1626 in a low-profile uninflated state. Figures 16C and 16D show a front view and a side view, respectively, of the dissection device 1600 with balloon 1626 in an inflated configuration. The balloon 1626 can be inflated to enlarge the space S into a dissection pocket DP of a desired size, for example a size appropriate to function as an autologous vein valve. The balloon 1626 may be inflated once or more than once, as appropriate for the procedure. After completion of the balloon dissection step, the dissection device 1600 can be deflated and removed. In this embodiment, the cutting element is a separate cutting device which is inserted into dissection pocket DP after the dissection device 1600 is removed. A suitable cutting device may be exchanged for the dissection device to cut the opening to create a pocket mouth M. This final step is necessary to transform the dissected pocket into a functioning valve. An embodiment of a suitable cutting device is shown in Figure 14.

An exemplary method of valve formation using valve creation assemblies that include separate dissection and cutting devices is now described. Any combination of suitable dissection and cutting devices can comprise a valve creation assembly according to the present technology. For example, the cutting device 1460 described in Figure 14 could be interchanged with the inflatable dissection device 1600 shown in Figure 16 or the dissection device 1300 shown in Figure 13 to carry out the dissection and cutting steps of valve formation. First, a dissection device is delivered through the opening O in vessel wall W and into the space S. Once the dissection device is positioned within the space S, the expandable member 22 is collapsed, and the support assembly is pulled back to provide more area in the vessel for the valve formation step. The dissection device of the valve creation assembly 17 is then actuated to separate tissue at the periphery PE of the space S. The enlarged space S forms a dissection pocket DP having a predetermined size and shape and extending along a dissection plane P within the vessel wall W. After a first expansion, the dissection device can be collapsed, translated within the space S, and then re-expanded one or more times in order to form a dissection pocket DP with the desired geometry. To transform the dissection pocket DP into a valve leaflet L a cutting device is used to cut the tissue at the proximal edge E of the dissection pocket DP adjacent the opening O. For example, the cutting device can cut the vessel wall tissue at the edge of the dissection pocket DP that extends laterally away from the opening O, as indicated by arrows A in Figure 3C. The cutting device may be interchanged with the dissection device in order to perform the cutting step, or may be provided to the treatment area in addition to the dissection device. One method of interchanging a dissection device for a cutting device is to advance a sleeve over the dissection device, removing the dissection device from the sleeve, and using the sleeve to deliver a cutting device to the dissection pocket DP.

### 5.0 Representative Methods for Using the Valve Formation Systems of the Present Technology

Described now are exemplary methods for intravascular creation of valve leaflets within blood vessels. Figures 17A-17O show the steps of an exemplary procedure. Reference is also made to anatomical structures in Figures 3A-3F.

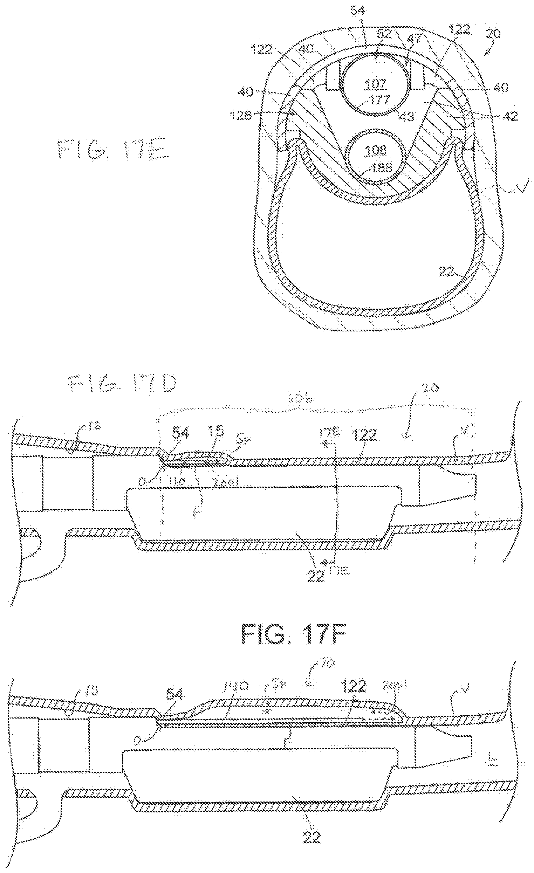

In a first step, access to the target vessel is obtained with a 0.035" or 0.038" guidewire using standard interventional techniques. The catheter assembly 11 is then positioned over the guidewire and inserted into the target vessel near the intended treatment area, using the guidewire/visualization lumen 108 (visible in Figure 17D) in the catheter to guide the catheter over the guidewire. Next, a visualization device such as an intravascular ultrasound (IVUS) catheter is exchanged for the guidewire in the guidewire/visualization lumen 108 and advanced into the target vessel distal to the catheter assembly 11. Figure 17A shows the support assembly 20 of catheter assembly 11 positioned within a target vessel during visualization of the local blood vessel anatomy. For example, a visualization device (e.g., an intravascular ultrasound ("IVUS") catheter and/or other suitable intravascular visualization devices) (not visible in Figure 17A) can be advanced through the second lumen 108 (see Figure 4) of the support assembly 20 to the treatment site. The visualization device can be used to identify a suitable site for the procedure, including identification of vessel features such as side branches, diseased sections, and/or existing leaflets. For example, a site with a minimal amount of these anatomical structures is preferred if the procedure is to create a new autologous valve. The visualization device can emit a visualization signal S, for example an ultrasound signal from an intravascular ultrasound (IVUS) catheter. Translation of the visualization transducer in the second lumen 108 allows a signal to be obtained along the length of the support assembly 20. Once a suitable target site for a procedure is identified, the support assembly 20 is rotated as required to position the device at the desired orientation at the target site, as shown in Figure 17B. The rotation can be accomplished, for example, via a rotation actuator 534 on the handle assembly 30 (see Figure 8A and 8B).

As shown in Figure 17C, the expandable member 22 may be expanded to urge the vessel wall V towards to the slanted surface 54 and surface 122 of the support assembly 20 and/or conform the vessel wall V to the slanted surface 54 and surface 122. The expandable member 22 can be expanded, for example, by injecting fluid into the expandable member 22 from an injection port (such as the third connector 536 shown in Figures 8A and 8B) and via one or more inflation lumens 116 (see Figure 4). Using the visualization device positioned within the visualization lumen 108 (see Figure 4), the user can visualize the expandable member 22 while it expands and can determine the appropriate level of expansion to achieve the desired vessel wall conformation against the slanted surface 54 and surface 122.

As shown in Figure 17D, the tissue penetration assembly 15 may then be advanced through the device lumen 107 (see Figure 17E), out the exit port 124 on the slanted surface 54, and towards the tissue apposed against the slanted surface 54. Figure 17E is a cross-sectional end view taken along line 17E-17E in Figure 17D, and is similar to a cross sectional view obtained by the visualization device when positioned along the second portion 106 of the support assembly 20. As the tissue penetration assembly 15 is advanced the tissue penetrating element 110 punctures the inner surface IS of the vessel wall V to form an opening O in the vessel wall V. The location and angle of the initial puncture into the inner surface of the vessel wall V is determined by the location of the device lumen 107 with respect to the slanted surface 54 and the surfaces 122, the location of the tissue penetrating element 110 within the device lumen 107, and the bevel geometry at the distal face 160 (see Figure 9A) of the tissue penetrating element 110. These locations and the bevel geometry of the tissue penetrating element 110 are configured to enable access the vessel wall V at predetermined depth along the thickness of the wall to separate a very thin layer of tissue F from the vessel wall In some embodiments, the thickness of the layer and/or depth of the vessel wall accessed is between about 0.006" and about 0.012". In some embodiments, the tissue penetration assembly 15 is advanced between 20 mm and 40 mm.

The user may continue to advance the tissue penetration assembly 15 through the opening O, along the surfaces 122, and in a direction generally parallel to a longitudinal axis of the vessel V to create a space Sp within the layers of the vessel wall W. As shown in Figure 17F, in some embodiments the cover tube 140 may be advanced over the penetrating element 110 after the penetrating element 110 has punctured the inner surface IS of the vessel wall V but before the puncture element 110 has advanced too far (e.g., near the distal end of the surfaces 22) between vessel wall layers. For example, the cover tube 140 can cover the penetrating element 110 after the penetrating element has advanced between about 3 mm and about 15 mm, and in some embodiments, between about 5 mm and about 12 mm, and in some embodiments, between about 3 mm and about 10 mm. In some embodiments, the cover tube 140 can cover the penetrating element 110 after the penetrating element has advanced between about 5 mm and about 10 mm. The cover tube 140 can reduce the risk of the penetrating element 110 penetrating outside the desired tissue layer, for example back into the vessel lumen L or outside the vessel wall. In some embodiments, the total advancement of tissue penetration assembly 15 is between about 20 mm and about 40 mm. In some embodiments, the total advancement of the tissue penetration assembly 15 is between about 25 mm and about 35 mm.

In some embodiments, a pressurized fluid source is connected to the tissue penetration assembly 15 to provide outward hydrostatic pressure (e.g., by ejecting fluid 2001) in the created space Sp during advancement of the tissue penetration assembly 15 through the vessel wall layers. Using fluid pressure can widen the space Sp for subsequent procedure steps while reducing the risk of the needle penetrating outside the targeted tissue layer.

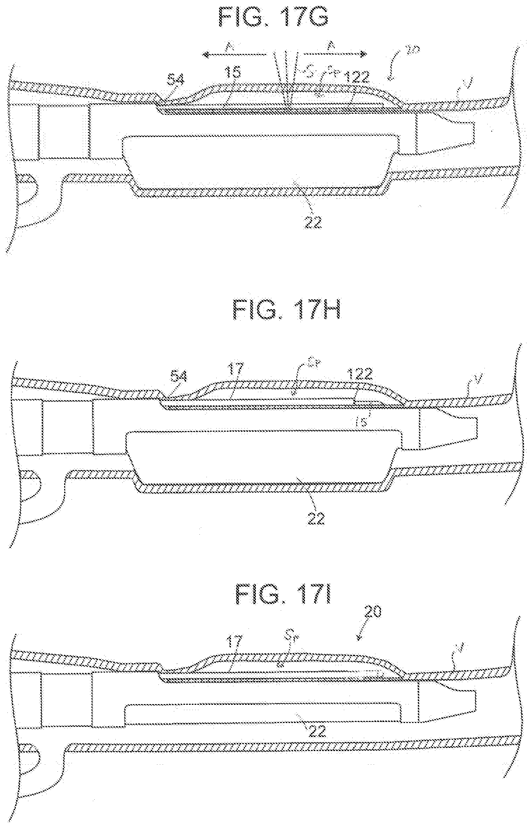

As depicted in Figure 17G, after the tissue penetration assembly 15 has advanced the desired amount within the vessel wall V, the visualization device can be used to assess the space Sp. Translation of the visualization device in the second lumen 108 (Figure 17E) along the space Sp (shown by the arrows A in Figure 17G) can allow for assessment of the entire space Sp.

Next, as shown in Figure 17H, the valve creation assembly 17 is advanced through the device lumen 107 (Figure 17E) over the tissue penetration assembly 15 and into the space Sp. During this period, pressurized fluid flow (not shown) may continue through the tissue penetration assembly 15 to maintain a pressurized space Sp and minimize risk of vessel perforation during advancement of the valve creation assembly 17. In some embodiments, the tissue penetration assembly 15 may be removed from the space Sp prior to insertion of the valve creation assembly 17. The valve creation assembly 17 may be advanced the same amount as the tissue penetration assembly 15, or may be advanced more or less than the tissue penetration assembly 15. In some embodiments, the valve creation assembly 17 is advanced between 20 mm and 40 mm. Once the valve creation assembly 17 is situated in space Sp, the expandable member 22 may be collapsed, as illustrated in Figure 17I, and the support assembly 20 may be withdrawn to allow for more space while forming the valve In some embodiments, the support assembly 20 is withdrawn between about 4 cm and about 7 cm. In some embodiments, the support assembly 20 is withdrawn between about 5 cm and about 6 cm. The tissue penetration assembly 15 may also be withdrawn while withdrawing the support assembly 20 and/or prior to expanding the valve creation assembly 17 to form the valve. In some embodiments, the user may advance a visualization device 2000 (the same or different visualization device referred in Figures 17A-17G) to the treatment site after the support assembly 20 has been withdrawn but before the valve creation assembly 17 is expanded to further assess the space Sp in preparation for forming the valve.

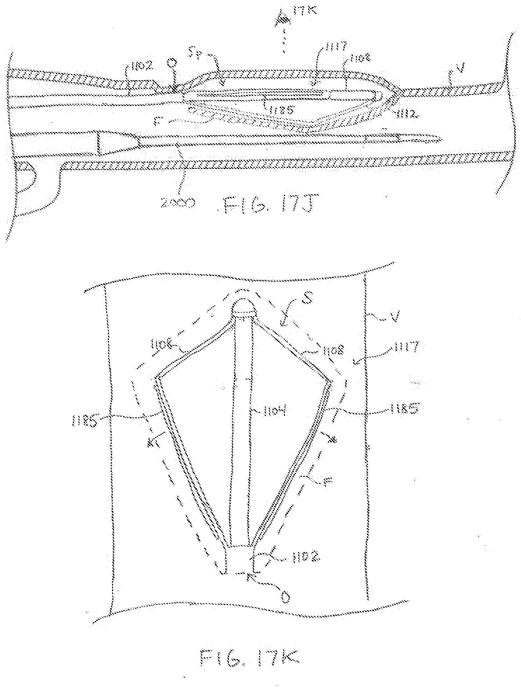

The user may then utilize the valve creation assembly 17 to create a valve. In some embodiments, such as those depicted by the method shown in Figures 17J-17N, the valve creation assembly 17 may be actuated (as detailed above with reference to Figures 11A-16D) and translated (proximally and/or distally) in the space Sp one or more times to create a dissection pocket DP and a mouth M, which together form the valve. The method for forming a valve utilizing a valve creation assembly 17 is described in Figures 17K-17M with reference to valve creation assembly 1117 shown in Figures 11A and 11B. It will be appreciated, however, that any of the valve creation assemblies 1117 disclosed herein may be used with any of the methods disclosed herein for forming a valve.

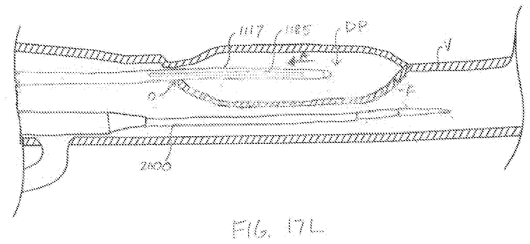

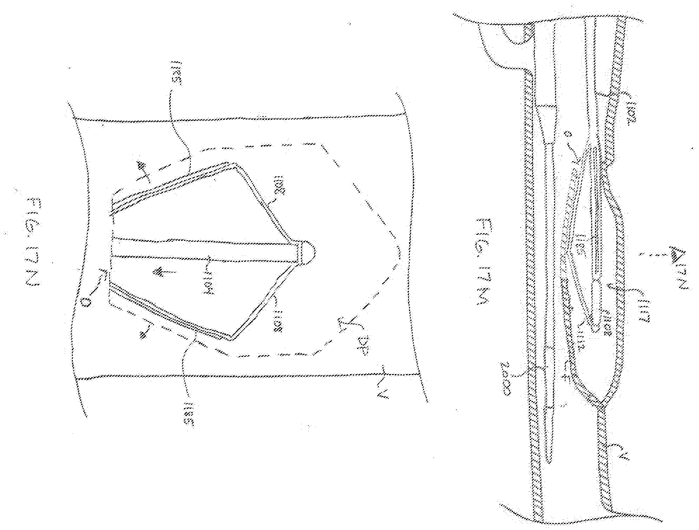

Figure 17J shows the valve creation assembly 1117 with the dissection arms 1108 and the tension arm 1112 expanding within the space Sp to create a dissection pocket DP. Figure 17K is a top view of the valve creation assembly 1117 within the dissection pocket DP as shown in Figure 17J. Figure 17L shows the valve creation assembly 1117 collapsed and being translated proximally (i.e., in the direction indicated by arrow A) to a position where at least a portion of the cutting elements 1185 are aligned with the opening O. As shown in Figures 17M and N, the expansion and/or pulling back proximally of the valve creation assembly 1117 in an expanded state cuts the opening O to create mouth M. The valve creation assembly 17 is translated proximally while the dissection arms 1108 (including cutting elements 1185) are in a deployed state. Expansion of the dissection arms 1108 and/or translation of assembly 1117 causes the cutting elements 1185 to cut tissue at the edge of the opening O to form the mouth M. The resultant structure functions as a valve leaflet. The tension arm 1112 can provide tension to the tissue to ease in the cutting of the vessel wall tissue.

In those embodiments where the dissection arms 1108 and/or cutting elements 1185 are angled distally (similar to the embodiment shown in Figure 12),cutting the tissue at the opening O may include pushing the valve creation assembly distally. In some embodiments, the valve creation assembly 17 can include separate dissection and cutting devices. In an example not part of the present invention, the dissection device can comprise an inflatable structure that is inflated to create the dissection pocket DP, or a mechanical structure without cutting elements which is expanded to create dissection pocket DP. In these embodiments, the dissection device is exchanged for a cutting device that cuts the opening to form mouth M.

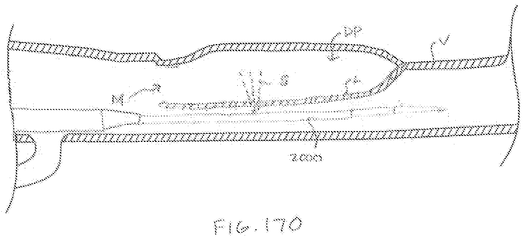

In some embodiments, the valve can be tested with fluid and contrast to visualize the function and mobility of the leaflet L via a flush lumen in catheter assembly 11, or through an introducer sheath, as depicted in Figure 17O.

If desired, a secondary device may be inserted into the formed leaflet to increase the size of the leaflet and/or urge the leaflet to take a shape which more easily moves away from the wall during normal blood flow and thus more likely impedes retrograde flow as is its intent. For example, an expandable catheter such as a balloon catheter may be directed into the formed valve leaflet under fluoroscopic and/or IVUS imaging and inflated. An example would be a PTA balloon catheter or a Fogarty thrombectomy balloon catheter. Other balloon catheter devices or mechanically expanding devices may be used. In another example, the catheter assembly 11 is directed into the leaflet and positioned such that the balloon 22 is toward the lumen. The balloon 22 is inflated to further expand the leaflet.

In some embodiments, a secondary device may comprise a clip and clip insertion tool which secures the formed valve leaflet into a specific modality. Examples of suitable devices include those disclosed in U.S. Patent No. 9,545,289, filed February 25, 2011, and U.S. Patent Application No. 13/450, 432, filed April 18, 2012.

If desired, the support assembly 20 can be rotated 180 degrees to form a leaflet on the opposite side. The resultant two opposing leaflets form a bicuspid valve in the vessel. If desired, additional valves may be formed at different target sites in the same vessel by moving the catheter assembly 11 to position support assembly 20 at a new target site.

### 7.0 Conclusion

This disclosure is not intended to be exhaustive or to limit the present technology to the precise forms disclosed herein. Although specific embodiments are disclosed herein for illustrative purposes, various equivalent modifications are possible without deviating from the present technology, as those of ordinary skill in the relevant art will recognize. In some cases, well-known structures and functions have not been shown and/or described in detail to avoid unnecessarily obscuring the description of the embodiments of the present technology. Although steps of methods may be presented herein in a particular order, in alternative embodiments the steps may have another suitable order. Similarly, certain aspects of the present technology disclosed in the context of particular embodiments can be combined or eliminated In some embodiments. Furthermore, while advantages associated with certain embodiments may have been disclosed in the context of those embodiments, other embodiments can also exhibit such advantages, and not all embodiments need necessarily exhibit such advantages or other advantages disclosed herein to fall within the scope of the present technology. Accordingly, this disclosure and associated technology can encompass other embodiments not expressly shown and/or described herein.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or ( c) any combination of the items in the list. Additionally, the terms "comprising" and the like are used throughout this disclosure to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various elements. It should be understood that such terms do not denote absolute orientation. Reference herein to "one embodiment," "an embodiment," or similar formulations means that a particular feature, structure, operation, or characteristic described in connection with the embodiment can be included in at least one embodiment of the present technology. Thus, the appearances of such phrases or formulations herein are not necessarily all referring to the same embodiment. Furthermore, various particular features, structures, operations, or characteristics may be combined in any suitable manner in one or more embodiments.

## Claims

1. A system (10) for controlled dissection of a blood vessel wall (W), the system (10) comprising:
a catheter assembly (11) comprising (a) an elongated shaft (12) having a proximal portion (14) and a distal portion (16) configured to be intravascularly delivered to a treatment site within a blood vessel lumen, (b) a support assembly (20) at the distal portion (16) of the elongated shaft (12), and (c) a lumen (107) extending from the proximal portion (14) to an opening (52) along the support assembly (20);
a valve creation assembly (1117) configured to be slidably received within the lumen (107) of the catheter assembly (11) and exit the lumen (107) through the opening (52), the valve creation assembly (1117) configured to be positioned within a blood vessel wall (W), wherein the valve creation assembly (1117) includes-an outer shaft (1102),
an inner member (1104) extending through the outer shaft (1102),
a dissection arm (1108) carried by the outer shaft (1102) and configured to expand radially outwardly away from the outer shaft (1102) when the inner member (1104) moves proximally relative to the outer shaft (1102),
a tensioning arm (1112) configured to extend radially outwardly from the inner member (1104) within a plane at a non-zero angle with respect to the plane within which the dissection arm (1108) expands; and
a handle assembly (30) coupled to the elongated shaft (12) and the valve creation assembly (1117), the handle assembly (30) including an actuator (533) coupled to the outer shaft (1102) of the valve creation assembly (1117), wherein movement of the actuator (533) relative to the elongated shaft (12) and/or handle assembly (30) causes the valve creation assembly (1117) to expand and collapse.

2. The system of claim 1 wherein translation of the actuator (533) by a user causes the valve creation assembly (1117) to expand and collapse.

3. The system of claim 1 or 2 wherein the handle assembly (30) further comprises a stop coupled to the actuator (533), wherein the stop limits an expansion size of the valve creation assembly (1117).

4. The system of claim 3, wherein the stop is an adjustable stop configured to be manipulated by the user to control a maximum expansion of the valve creation assembly (1117).

5. The system of any one of claims 1-4 wherein the support assembly (20) includes an expandable member (22) configured to expand into apposition with the blood vessel wall at the treatment site, thereby conforming the vessel wall at the treatment site to at least a portion of the support assembly (20).

6. The system of any one of claims 1-5 wherein the non-zero angle of the plane in which the tensioning arm (1112) expands is from about 40 degrees to about 90 degrees.

7. The system of any one of claims 1-6 wherein the dissection arm (1108) is a first dissection arm, and the valve creation assembly (1117) further includes a second dissection arm (1108) carried by the outer shaft (1102) and configured to expand radially outwardly away from the outer shaft (1102).

8. The system of any one of claims 1-7 wherein:
the valve creation assembly (1117) has a central lumen (1187); and
the system further comprises a tissue penetrating assembly (15) configured to be slidably disposed within the central lumen (1187) of the valve creation assembly and further configured to extend through the opening (52) of the catheter assembly (11) to penetrate the blood vessel wall (W) at a predetermined depth.

9. The system of claim 8 wherein the tissue penetrating assembly (15) comprises:
a tissue penetrating element (110) ; and
additionally comprises a cover tube (140) slidaby disposed around the tissue penetrating element (111).

10. The system of claim 9 wherein:
the handle assembly (30) comprises a further actuator (531) and the further actuator (531) is a knob which is configured to translate, proximally and distally, both the tissue penetrating element (110) and the tubular cover (140) at the same time.

11. The system of claim 9 wherein:
the handle assembly (30) comprises a further actuator (531) and the further actuator (531) is a knob which is configured to translate, proximally and distally, the tissue penetrating element (110) and the tubular cover (140) separately.

12. The system of any one of claims 1-9 wherein the handle assembly (30) includes an additional actuator (532) which is configured to axially, proximally and distally, move the valve creation assembly (1117).

13. The system of claim 12 wherein the actuator (533) is movable while the additional actuator (532) is in any position of actuation and vice versa, thereby allowing the valve creation assembly (1117) to expand and collapse at any point while translating proximally or distally.

## Patentansprüche

1. System (10) zur kontrollierten Dissektion einer Blutgefäßwand (W), wobei das System (10) umfasst:
Eine Katheteranordnung (11) umfassend: (a) einen länglichen Schaft (12) mit einem proximalen Teil (14) und einem distalen Teil (16), konfiguriert intravaskulär zu einer Behandlungsstelle innerhalb eines Blutgefäßlumens gebracht zu werden, (b) eine Stützbaugruppe (20) am distalen Teil (16) des länglichen Schafts (12) und (c) ein Lumen (107), da sich vom proximalen Teil (14) zu einer Öffnung (52) entlang der Stützbaugruppe (20) erstreckt;
eine Ventilherstellungsbaugruppe (1117), die konfiguriert ist, verschiebbar innerhalb des Lumens (107) der Katheteranordnung (11) aufgenommen zu werden und das Lumen (107) durch die Öffnung (52) verlässt, wobei die Ventilherstellungsbaugruppe (1117) konfiguriert ist, innerhalb einer Blutgefäßwand (W) positioniert zu werden, wobei die Ventilherstellungsbaugruppe (1117) einschließt- einen äußeren Schaft (1102),
ein inneres Element (1104), das sich durch den äußeren Schaft (1102) erstreckt,
ein Dissektionsarm (1108), der vom äußeren Schaft (1102) getragen wird und konfiguriert ist, radial nach außen vom äußeren Schaft (1102) weg zu expandieren, wenn sich das innere Element (1104) proximal relativ zum äußeren Schaft (1102) bewegt, einen Spannungsarm (1112), der konfiguriert ist, sich radial vom inneren Element (1104) innerhalb einer Ebene in einem Winkel ungleich null mit Bezug auf die Ebene zu erstrecken, innerhalb welcher der Dissektionsarm (1108) expandiert; und
eine Griffbaugruppe (30), die an den länglichen Schaft (12) und die Ventilherstellungsbaugruppe (1117) gekoppelt ist, wobei die Griffbaugruppe (30) ein Betätigungselement (533) einschließt, das an den äußeren Schaft (1102) der Ventilherstellungsbaugruppe (1117) gekoppelt ist, wobei die Bewegung des Betätigungselements (533) relativ zum länglichen Schaft (12) und/oder der Griffbaugruppe (30) bewirkt, dass die Ventilherstellungsbaugruppe (1117) expandiert und kollabiert.

2. System nach Anspruch 1, wobei translatorische Bewegung des Betätigungselements (533) durch einen Benutzer bewirkt, dass die Ventilherstellungsbaugruppe (1117) expandiert und kollabiert.

3. System nach Anspruch 1 oder 2, wobei die Griffbaugruppe (30) ferner einen an das Betätigungselement (533) gekoppelten Anschlag umfasst, wobei der Anschlag eine Expansionsgröße der Ventilherstellungsbaugruppe (1117) begrenzt.

4. System nach Anspruch 3, wobei der Anschlag ein verstellbarer Anschlag ist, der konfiguriert ist, vom Benutzer manipuliert zu werden, um eine maximale Expansion der Ventilherstellungsbaugruppe (1117) zu steuern.

5. System nach irgendeinem der Ansprüche 1-4, wobei die Stützbaugruppe (20) ein expandierbares Element (22) einschließt, das konfiguriert ist, in Apposition mit der Blutgefäßwand an der Behandlungsstelle zu expandieren, um dadurch die Blutgefäßwand an der Behandlungsstelle zumindest einem Teil der Stützbaugruppe (20) anzupassen.

6. System nach irgendeinem der Ansprüche 1-5, wobei der Winkel von ungleich null der Ebene, in welcher der Spannungsarm (1112) expandiert, von ca. 40 Grad bis ca. 90 Grad beträgt.

7. System nach irgendeinem der Ansprüche 1-6, wobei der Dissektionsarm (1108) ein erster Dissektionsarm ist, und die Ventilherstellungseinheit (1117) ferner einen zweiten Dissektionsarm (1108) einschließt, der vom äußeren Schaft (1102) getragen wird und konfiguriert ist, radial vom äußeren Schaft (1102) weg nach außen zu expandieren.

8. System nach irgendeinem der Ansprüche 1-7, wobei:
Die Ventilherstellungsbaugruppe (1117) ein mittiges Lumen (1187) aufweist; und das System ferner eine Gewebe penetrierende Baugruppe (15) umfasst, die konfiguriert ist, verschiebbar innerhalb des mittigen Lumens (1187) der Ventilherstellungsbaugruppe angeordnet zu sein, und ferner konfiguriert ist, sich durch die Öffnung (52) der Katheteranordnung (11) zu erstrecken, um die Blutgefäßwand (W) in einer vorbestimmten Tiefe zu penetrieren.

9. System nach Anspruch 8, wobei die Gewebe penetrierende Baugruppe (15) umfasst:
Ein Gewebe penetrierendes Element (110); und
zusätzlich ein Abdeckrohr (140) umfasst, das verschiebbar um das Gewebe penetrierende Element (111) angeordnet ist.

10. Das System nach Anspruch 9, wobei:
Die Griffbaugruppe (30) ein weiteres Betätigungselement (531) umfasst und das weitere Betätigungselement (531) ein Knopf ist, der konfiguriert ist, sowohl das Gewebe penetrierende Element (110) als auch die rohrförmige Abdeckung (140) gleichzeitig proximal und distal translatorisch zu bewegen.

11. System nach Anspruch 9, wobei die Griffbaugruppe (30) ein weiteres Betätigungselement (531) umfasst und das weitere Betätigungselement (531) ein Knopf ist, der konfiguriert ist, das Gewebe penetrierende Element (110) und die rohrförmige Abdeckung (140) separat proximal und distal translatorisch zu bewegen.

12. System nach irgendeinem der Ansprüche 1-9, wobei die Griffbaugruppe (30) ein zusätzliches Betätigungselement (532) einschließt, das konfiguriert ist, die Ventilherstellungsbaugruppe (1117) axial, proximal und distal zu bewegen.

13. System nach Anspruch 12, wobei das Betätigungselement (533) beweglich ist, während sich das zusätzliche Betätigungselement (532) in irgendeiner Betätigungsposition und umgekehrt befindet, womit der Ventilherstellungsbaugruppe (1117) ermöglicht wird, während proximaler oder distaler translatorischer Bewegung zu expandieren oder zu kollabieren.

## Revendications

1. Un système (10) permettant une dissection régulée d'une paroi de vaisseau sanguin (W), le système (10) comprenant :
un ensemble cathéter (11) comprenant (a) un arbre allongé (12) ayant une partie proximale (14) et une partie distale (16), configuré pour être amené de manière intravasculaire à un site de traitement dans une lumière de vaisseau sanguin, (b) un ensemble support (20) à la partie distale (16) de l'arbre allongé (12) et (c) une lumière (107) s'étendant de la partie proximale (14) à une ouverture (52) le long de l'ensemble support (20) ;
un ensemble création de valve (1117) configuré pour être reçu de manière coulissante dans la lumière (107) de l'ensemble cathéter (11) et pour sortir de la lumière (107) à travers l'ouverture (52), l'ensemble création de valve (1117) étant configuré pour être positionné dans une paroi de vaisseau sanguin (W), dans lequel l'ensemble création de valve (1117) inclut un arbre extérieur (1102),
un élément intérieur (1104) s'étendant à travers l'arbre extérieur (1102),
un bras de dissection (1108) porté par l'arbre extérieur (1102) et configuré pour se dilater radialement vers l'extérieur en s'écartant de l'arbre extérieur (1102) quand l'élément intérieur (1104) se déplace proximalement par rapport à l'arbre extérieur (1102),
un bras de tensionnement (1112) configuré pour s'étendre radialement vers l'extérieur depuis l'élément intérieur (1104) dans un plan à un angle qui n'est pas zéro par rapport au plan dans lequel le bras de dissection (1108) se dilate ; et
un ensemble poignée (30) couplé à l'arbre allongé (12) et à l'ensemble création de valve (1117), l'ensemble poignée (30) incluant un actionneur (533) couplé à l'arbre extérieur (1102) de l'ensemble création de valve (1117), dans lequel le déplacement de l'actionneur (533) par rapport à l'arbre allongé (12) et/ou à l'ensemble poignée (30) amène l'ensemble création de valve (1117) à se dilater et se rétracter.

2. Le système selon la revendication 1, dans lequel la translation de l'actionneur (533) par un utilisateur amène l'ensemble création de valve (1117) à se dilater et se rétracter.

3. Le système selon la revendication 1 ou 2, dans lequel l'ensemble poignée (30) comprend en outre une butée couplée à l'actionneur (533), dans lequel la butée limite une dimension de dilatation de l'ensemble création de valve (1117).

4. Le système selon la revendication 3, dans lequel la butée est une butée réglable configurée pour être manipulée par l'utilisateur pour réguler une dilatation maximale de l'ensemble création de valve (1117).

5. Le système selon l'une quelconque des revendications 1-4, dans lequel l'ensemble support (20) inclut un élément dilatable (22) configuré pour se dilater en apposition avec la paroi de vaisseau sanguin au site de traitement, conformant ainsi la paroi de vaisseau au site de traitement à au moins une partie de l'ensemble support (20).

6. Le système selon l'une quelconque des revendications 1-5, dans lequel l'angle qui n'est pas zéro par rapport au plan dans lequel le bras de tensionnement (1112) se dilate, est compris entre environ 40 degrés et environ 90 degrés.

7. Le système selon l'une quelconque des revendications 1-6, dans lequel le bras de dissection (1108) est un premier bras de dissection, et l'ensemble création de valve (1117) inclut en outre un deuxième bras de dissection (1108) porté par l'arbre extérieur (1102) et configuré pour se dilater radialement vers l'extérieur en s'écartant de l'arbre extérieur (1102).

8. Le système selon l'une quelconque des revendications 1-7, dans lequel :
l'ensemble création de valve (1117) a une lumière centrale (1187) ; et
le système comprend en outre un ensemble pénétration de tissu (15) configuré pour être disposé de manière coulissante dans la lumière centrale (1187) de l'ensemble création de valve et configuré en outre pour s'étendre à travers l'ouverture (52) de l'ensemble cathéter (11) pour pénétrer dans la paroi de vaisseau sanguin (W) à une profondeur prédéterminée.

9. Le système selon la revendication 8, dans lequel l'ensemble pénétration de tissu (15) comprend :
un élément pénétration de tissu (110) ; et
en plus un tube de fermeture (140) disposé de manière coulissante autour de l'élément pénétration de tissu (111).

10. Le système selon la revendication 9, dans lequel :
l'ensemble poignée (30) comprend un autre actionneur (531) et l'autre actionneur (531) est un bouton qui est configuré pour translater, proximalement et distalement, à la fois l'élément de pénétration de tissu (110) et la fermeture tubulaire (140) en même temps.

11. Le système selon la revendication 9, dans lequel
l'ensemble poignée (30) comprend un autre actionneur (531) et ledit autre actionneur (531) est un bouton qui est configuré pour translater, proximalement et distalement, l'élément pénétration de tissu (110) et la fermeture tubulaire (140) séparément.

12. Le système selon l'une quelconque des revendications 1-9, dans lequel l'ensemble poignée (30) inclut un actionneur supplémentaire (532) qui est configuré pour déplacer l'ensemble création de valve (1117) axialement, proximalement et distalement.

13. Le système selon la revendication 12, dans lequel l'actionneur (533) peut être déplacé alors que l'actionneur supplémentaire (532) est dans une position d'actionnement quelconque et vice versa, permettant ainsi à l'ensemble création de valve (1117) de se dilater et se rétracter à un point quelconque lors de la translation proximale ou distale.
